# EUROPEAN PATENT APPLICATION

(11) **EP 2 905 024 A1**
(43) Date of publication of application: **12.08.2015**
(21) Application number: 14382046.2
(22) Date of filing: 07.02.2014
(51) Int. Cl.: A61K 31/519, C07D 235/02, A61P 31/14

(54) **Pyrido[2,3-d]pyrimidine-7(8H)-one derivatives for the treatment of infections caused by Flaviviridae**

(71) Applicant: Institut Quimic De Sarriá Cets, Fundació Privada, 08017 Barcelona (ES)
(72) Inventor: Borrell Bilbao, José Ignacio, E-08017 Barcelona (ES); Teixido Closa, Jordi, E-08017 Barcelona (ES); Camarasa Navarro, Marta, E-08017 Barcelona (ES); Puig De La Bellacasa Cazorla, Raimon, E-08017 Barcelona (ES); Martínez De La Sierra, Miguel Angel, E-08916 Badalona-Barcelona (ES); Franco Cirera, Sandra, E-08916 Badalona-Barcelona (ES); Badia Córcoles, Roger, E-08916 Badalona-Barcelona (ES); Clotet Sala, Bonaventura, E-08916 Badalona-Barcelona (ES); Esté Araque, José, E-08916 Badalona-Barcelona (ES)
(74) Representative: ABG Patentes, S.L.

(57) **Abstract**

The present invention is related to pyrido[2,3-*d*]pyrimidine derivatives, to pharmaceutical compositions comprising them, and to their use in the treatment and/or prevention of infections caused by viruses of the *Flaviriade* family, in particular, hepatitis C virus.

## Description

### FIELD OF THE INVENTION

The present invention relates to pyrido[2,3-*d*]pyrimidine-7(8H)-one derivatives, to pharmaceutical compositions comprising them, and to their use in the treatment and/or prevention of infections caused by viruses of the *Flaviriade* family, in particular hepatitis C.

### BACKGROUND OF THE INVENTION

Hepatitis C virus (HCV) belongs to the genus *Hepacivirus,* a member of the family *Flaviviridae.* HCV is a positive-sense single-stranded RNA virus of approximately 9.6 Kb. Based on genetic differences between HCV isolates, the hepatitis C virus species is classified into seven genotypes (1-7) with several subtypes within each genotype. Genotypes differ by 30-35% of the nucleotide sites over the complete genome. Subtypes 1a and 1b are found worldwide and cause 60% of hepatitis C cases.

Hepatitis C is an infectious disease affecting primarily the liver, caused by the HCV. ACV has infected an estimated 150-200 million people worldwide. The virus persists in the liver in about 85% of those infected.

The standard treatment for hepatitis C is based on a combination of (pegylated) interferon-alpha (PEG-IFN-α) plus ribavirin for 24 or 48 weeks, depending on the HCV genotype. This combination therapy yields a sustained virologic response in more than 40% of patients infected by genotype 1 viruses and about 80% of those infected by genotypes 2 and 3. Besides the limited efficacy on HCV type 1, this combination therapy has significant side effects and is poorly tolerated in many patients.

The most common side effects that may occur after treatment with PEG-IFN-α are: nausea, diarrhea, fever, chills, muscle and joint pain, difficulty concentrating, thyroid disorders, hair loss, insomnia, irritability, mild to severe depression, and rarely, suicidal thoughts. Other serious side effects that may be manifested are bone marrow toxicity, cardiovascular disorders, hypersensitivity disorders, endocrine disorders, pulmonary disorders, colitis, pancreatitis and ophthalmologic disorders. PEG-IFN-α can cause neuropsychiatric, autoimmune, ischemic and infectious fatal.

With respect to ribavirin, anemia is often the most frequent side effect during the treatment of hepatitis C. Ribavirin can also cause decreased white blood cells and platelets.

Different pyrido[2,3-d]pyrimidine compounds have been proposed for treating a variety of diseases (see for example US2004/0038856, WO04/020584, WO2003/059913, JP2003321472, WO 95/19774, US3873545, WO2006/258686, WO2011/090666 and WO2011/044535).

Additionally, WO2005/105097 discloses the use of pyridopyrimidine derivatives in the treatment of inflammatory diseases, neurodegenerative disorders, autoimmune diseases, neuroimmunological diseases, autoimmune diseases, diabetes, transplant rejection, infective diseases, prion diseases, cardiovascular diseases and disorders and WO2006/120252 and US2011/0123493 describe the use of pyrido[2,3-d]pyrimidines for the treatment of HCV.

There is still a need for clinically effective agents for the treatment of infections caused by viruses of the *Flaviviridae* family, such as hepatitis C, Dengue fever, Japanese encephalitis, Kyasanur Forest disease, Murray Valley encephalitis, St. Louis encephalitis, Tick-borne encephalitis, West Nile encephalitis and yellow fever, specially by the hepatitis C virus (HCV), which may improve current therapeutic strategies, in particular which may reduce unwanted side effects of current drugs.

The inventors have surprisingly found that new pyrido[2,3-d]pyrimidine derivatives of formula (I) are capable of inhibiting HCV. This discovery makes these compounds promising candidates for the treatment of infections caused by viruses of the *Flaviviridae* family, such as hepatitis C, Dengue fever, Japanese encephalitis, Kyasanur Forest disease, Murray Valley encephalitis, St. Louis encephalitis, Tick-borne encephalitis, West Nile encephalitis and yellow fever, in particular for the treatment of hepatitis C.

### SUMMARY OF THE INVENTION

In a first aspect, the present invention relates to a compound of formula (I): 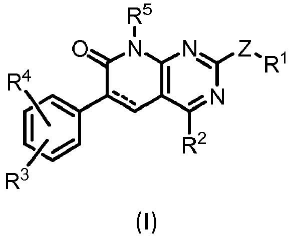 wherein
R¹ is selected from the group consisting of
   - H;
   - C₆-C₁₄ aryl optionally substituted with one, two or three substituents independently selected from the group consisting of C₁-C₆ alkyl, C₁-C₆ alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl; and
   - C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆ alkyl;
R² is selected from the group consisting of H; -NR⁶R⁷; and -OR⁸;
   where R⁶, R⁷ and R⁸ are independently selected from the group consisting of H, C₁-C₆ alkyl and C₁-C₆ alkyl-O-C₁-C₆ alkylene, and aromatic, saturated or partially saturated 3- to 8-membered monocyclic or 5- to 12-membered bicyclic heterocyclyl which contains from 1 to 4 heteroatoms in the ring system independently selected from the group consisting of N, O, and S, and said heterocyclyl being optionally substituted with C₁-C₆alkyl;
   or R⁶ and R⁷ together with the nitrogen atom to which they are attached form a saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl;
R³ and R⁴ are independently selected from the group consisting of H, F, Cl, Br, SO₂NH₂ and CF₃;
   or R³ and R⁴ together with the phenyl ring to which they are attached, form a C₉-C₁₄ aryl optionally substituted with one, two or three substituents independently selected from the group consisting of H, F, Cl, Br and CF₃;
R⁵ is selected from the group consisting of
   - H;
   - C₁-C₆ alkyl optionally substituted with one, two or three substituents independently selected from the group consisting of hydroxy; C₁-C₆ alkoxy, C₁-C₆ alkylthio, di(C₁-C₆alkyl)amino, C₁-C₆alkoxycarbonyl, cyano, and F;
   - C₃-C₈ cycloalkyl optionally substituted with one, two or three substituents independently selected from the group consisting of hydroxy; C₁-C₆ alkoxy, C₁-C₆ alkylthio, C₁-C₆alkoxycarbonyl, di(C₁-C₆alkyl)amino, cyano, and F;
   - C₃-C₈ cycloalkyl-C₁-C₃ alkylene, wherein the cycloalkyl is optionally substituted with one, two or three substituents independently selected from the group consisting of hydroxy; C₁-C₆ alkoxy, C₁-C₆ alkylthio, C₁-C₆ alkoxycarbonyl, di(C₁-C₆ alkyl)amino, cyano, and F;
   - C₆-C₁₄ aryl optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylsulfonyl, C₁-C₆ alkylsulfinyl di(C₁-C₆ alkyl)aminocarbonyloxy, di(C₁-C₆ alkyl)amino- C₁-C₆ alkyleneoxy, C₁-C₆ alkoxycarbonyl, and C₁-C₆alkoxy-C₁-C₆alkyleneoxy;
   - C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆alkoxycarbonyl, and C₁-C₆alkoxy-C₁-C₆alkyleneoxy;
   - 3- to 8-membered monocyclic or 5- to 12-membered bicyclic saturated or partially saturated heterocyclyl which contains from 1 to 4 heteroatoms in the ring independently selected from the group consisting of N, O, and S, said heterocyclyl being optionally substituted with one, two or three substituents independently selected from the group consisting of hydroxy; C₁-C₆ alkoxy, C₁-C₆ alkylthio, C₁-C₆alkoxycarbonyl, di(C₁-C₆alkyl)amino, cyano, and F;
   - heterocyclyl-C₁-C₃ alkylene, wherein the heterocyclyl is a 3- to 8-membered monocyclic or 5- to 12-membered bicyclic saturated or partially saturated ring system which contains from 1 to 4 heteroatoms in the ring independently selected from the group consisting of N, O, and S as defined above, said heterocyclyl being optionally substituted with one, two or three substituents independently selected from the group consisting of hydroxy; C₁-C₆ alkoxy, C₁-C₆ alkylthio, C₁-C₆alkoxycarbonyl, di(C₁-C₆alkyl)amino, cyano, and F;
   - 5- to 6-membered monocyclic or 8- to 14-membered bicyclic heteroaryl which contains from 1 to 4 heteroatoms in the ring system independently selected from the group consisting of N, O, and S, and wherein the heteroaryl is optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆ alkoxycarbonyl, and C₁-C₆alkoxy-C₁-C₆alkyleneoxy; and
   - heteroaryl-C₁-C₃ alkylene, wherein the heteroaryl is a 5- to 6-membered monocyclic or 8- to 14-membered bicyclic ring system which contains from 1 to 4 heteroatoms in the ring independently selected from the group consisting of N, O, and S, said heteroaryl being optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino- C₁-C₆ alkyleneoxy, C₁-C₆alkoxycarbonyl, and C₁-C₆alkoxy-C₁-C₆alkyleneoxy;
Z is selected from the group consisting of NH and O; and
   the dotted line represents a bond that may be present or not;
or stereoisomers, tautomers or pharmaceutically acceptable salts thereof,
for use in the treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever.

In a second aspect, the present invention relates to a compound of formula (II): 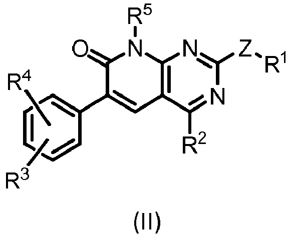 wherein
R¹ is selected from the group consisting of
   - H;
   - C₆-C₁₄ aryl optionally substituted with one, two or three substituents independently selected from the group consisting of C₁-C₆ alkyl, C₁-C₆ alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl; and
   - C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆ alkyl;
R² is selected from the group consisting of -NR⁶R⁷; and -OR⁸; where R⁶, R⁷ and R⁸ are as defined in the first aspect;
R³ and R⁴ are independently selected from the group consisting of H, F, Cl, Br, SO₂NH₂ and CF₃;
   or R³ and R⁴ together with the phenyl ring to which they are attached, form a C₉-C₁₄ aryl optionally substituted with one, two or three substituents independently selected from the group consisting of H, F, Cl, Br and CF₃;
R⁵ is selected from the group consisting of
   - H;
   - C₁-C₆ alkyl optionally substituted with one, two or three substituents independently selected from the group consisting of hydroxy; C₁-C₆ alkoxy, C₁-C₆ alkylthio, di(C₁-C₆alkyl)amino, C₁-C₆alkoxycarbonyl, cyano, and F;
   - C₃-C₈ cycloalkyl optionally substituted with one, two or three substituents independently selected from the group consisting of hydroxy; C₁-C₆ alkoxy, C₁-C₆ alkylthio, C₁-C₆alkoxycarbonyl, di(C₁-C₆alkyl)amino, cyano, and F;
   - C₃-C₈ cycloalkyl-C₁-C₃ alkylene, wherein the cycloalkyl is optionally substituted with one, two or three substituents independently selected from the group consisting of hydroxy; C₁-C₆ alkoxy, C₁-C₆ alkylthio, C₁-C₆ alkoxycarbonyl, di(C₁-C₆ alkyl)amino, cyano, and F;
   - C₆-C₁₄ aryl optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylsulfonyl, C₁-C₆ alkylsulfinyl di(C₁-C₆ alkyl)aminocarbonyloxy, di(C₁-C₆ alkyl)amino- C₁-C₆ alkyleneoxy, C₁-C₆ alkoxycarbonyl, and C₁-C₆alkoxy-C₁-C₆alkyleneoxy;
   - C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆alkoxycarbonyl, and C₁-C₆alkoxy-C₁-C₆alkyleneoxy;
   - 3- to 8-membered monocyclic or 5- to 12-membered bicyclic saturated or partially saturated heterocyclyl which contains from 1 to 4 heteroatoms in the ring independently selected from the group consisting of N, O, and S, said heterocyclyl being optionally substituted with one, two or three substituents independently selected from the group consisting of hydroxy; C₁-C₆ alkoxy, C₁-C₆ alkylthio, C₁-C₆alkoxycarbonyl, di(C₁-C₆alkyl)amino, cyano, and F;
   - heterocyclyl-C₁-C₃ alkylene, wherein the heterocyclyl is a 3- to 8-membered monocyclic or 5- to 12-membered bicyclic saturated or partially saturated ring system which contains from 1 to 4 heteroatoms in the ring independently selected from the group consisting of N, O, and S as defined above, said heterocyclyl being optionally substituted with one, two or three substituents independently selected from the group consisting of hydroxy; C₁-C₆ alkoxy, C₁-C₆ alkylthio, C₁-C₆alkoxycarbonyl, di(C₁-C₆alkyl)amino, cyano, and F;
   - 5- to 6-membered monocyclic or 8- to 14-membered bicyclic heteroaryl which contains from 1 to 4 heteroatoms in the ring system independently selected from the group consisting of N, O, and S, said heteroaryl being optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆ alkoxycarbonyl, and C₁-C₆alkoxy-C₁-C₆alkyleneoxy; and
   - heteroaryl-C₁-C₃ alkylene, wherein the heteroaryl is a 5- to 6-membered monocyclic or 8- to 14-membered bicyclic ring system which contains from 1 to 4 heteroatoms in the ring independently selected from the group consisting of N, O, and S, said heteroaryl being optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino- C₁-C₆ alkyleneoxy, C₁-C₆alkoxycarbonyl, and C₁-C₆alkoxy-C₁-C₆alkyleneoxy; and
Z is selected from the group consisting of NH and O;
or a stereoisomer, tautomer or pharmaceutically acceptable salt thereof,
with the proviso that when R³ and R⁴ are both Cl in *ortho* position with respect to the carbon atom bonded to the pyrido[2,3]pyrimidine-7(8H)one ring system, Z is NH, and R¹ is an optionally substituted phenyl, then R² is not NH₂.

In a third aspect, the present invention relates to a compound of formula (II) as defined in the second aspect or a stereoisomer, tautomer or pharmaceutically acceptable salt thereof, for use as a medicament.

In a fourth aspect, the present invention relates to a pharmaceutical composition comprising a compound of formula (II) as defined in the second aspect or a stereoisomer, tautomer or pharmaceutically acceptable salt thereof, and one or more pharmaceutically acceptable excipients.

### DESCRIPTION OF THE INVENTION

### Definitions

The term "alkyl" as employed herein alone or as part of another group designates a linear or branched saturated monovalent hydrocarbon chain containing from of one to six carbon atoms, preferably from one to three carbon atoms. Examples of alkyls are methyl, ethyl, propyl, 2-propyl, n-butyl, isobutyl, sec-butyl, tert-butyl, n-pentyl, isopentyl, sec-pentyl, tert-pentyl, neopentyl, and the like.

The term "alkylene" as employed herein alone or as part of another group designates a linear or branched saturated divalent hydrocarbon chain containing from of one to six carbon atoms, preferably from one to three carbon atoms. Examples of alkylene are methylene (-CH₂-), ethylene (-CH₂CH₂-), trimethylene (-CH₂CH₂CH₂-) or tetramethylene (-CH₂CH₂CH₂CH₂-), methylethylene, (-CH(CH₃)CH₂-), pentamethylene (-CH₂CH₂CH₂CH₂CH₂-), hexamethylene (-CH₂CH₂CH₂CH₂CH₂CH₂-) and the like.

The term "alkoxy" as employed herein alone or as a part of another group designates an alkyl group as defined above linked through oxygen, i.e. -O-alkyl, wherein the alkyl group may be substituted by an aryl group as defined below. Examples of which include methoxy, ethoxy, isopropoxy, tertbutoxy, benzyloxy, and the like.

The term "alkoxyalkyleneoxy" refers to a -O-alkylene-O-alkyl group, wherein alkyl and alkylene are as defined above. Examples of alkoxyalkyleneoxy are methoxymethoxy, ethoxymethoxy, isopropoxymethoxy, tertbutoxymethoxy, methoxyethoxy, ethoxyethoxy, isopropoxyethoxy, tertbutoxyethoxy and the like.

The term "alkoxycarbonyl" refers to a -C(O)O-alkyl group, wherein alkyl is as defined above. Examples of alkoxycarbonyl are methoxycarbonyl, ethoxycarbonyl, isopropoxycarbonyl, tertbutoxycarbonyl and the like.

The term "alkylamino" refers to a -NHalkyl group, wherein alkyl is as defined above. Examples of alkylamino are methylamino, ethylamino, isopropylamino, tertbutylamino and the like.

The term "alkylcarbonyl" refers to a -C(O)-alkyl group, wherein alkyl is as defined above. Examples of alkylcarbonyl are methylcarbonyl, ethylcarbonyl, isopropylcarbonyl, tertbutylcarbonyl and the like.

The term "alkylcarbonyloxy" refers to a -O-C(O)-alkyl group, wherein alkyl is as defined above. Examples of alkylcarbonyloxy are methylcarbonyloxy, ethylcarbonyloxy, isopropylcarbonyloxy, tertbutylcarbonyloxy and the like.

The term "alkylsulfinyl" refers to a -SO-alkyl group, wherein alkyl is as defined above. Examples of alkylsulfinyl are methylsulfinyl, ethylsulfinyl, isopropylsulfinyl, tertbutylsulfinyl and the like.

The term "alkylsulfonyl" refers to a -SO₂-alkyl group, wherein alkyl is as defined above. Examples of alkylsulfonyl are methylsulfonyl, ethylsulfonyl, isopropylsulfonyl, tertbutylsulfonyl and the like.

The term "alkylthio" as employed herein alone or as a part of another group designates an alkyl group as defined above linked through a sulfur atom, i.e. -S-alkyl, wherein the alkyl group may be substituted by an aryl group as defined below. Examples of which include methylthio, ethylthio, isopropylthio, tertbutylthio, benzylthio, and the like.

The term "amino" refers to a -NH₂ group.

The term "aryl" means a mono-, bi- or tricyclic aromatic hydrocarbon group, containing the number of atoms indicated in each case, which is comprised between six and fourteen carbon atoms in the ring portion, wherein the monocyclic ring is aromatic, and at least one of the rings in the bi- or tricyclic ring is aromatic. Representative examples include phenyl, naphthyl, phenanthryl, indenyl, indanyl, and the like.

The term "arylalkylene" refers to an alkylene group as defined above, substituted with an aryl group as defined above. Examples of arylalkylene are benzyl, phenethyl, phenylpropyl, and the like.

The term "carboxy" refers to a -COOH group.

The term "cyano" refers to a -CN group.

The term "cycloalkyl" is used in the present invention to designate a monocyclic or fused bicyclic, saturated or partially unsaturated (but not aromatic), monovalent hydrocarbon ring system of three to eight carbon ring atoms. Fused bicyclic hydrocarbon radical includes bridged ring systems. The term "cycloalkyl", as used in the present invention, also encompasses the above-mentioned ring systems wherein one or two ring carbon atoms are replaced by a -C(O)- or -C(S)- group. More specifically, the term cycloalkyl includes, but is not limited to, cyclopropyl, cyclobutyl, cyclopentyl, cyclohexyl, cyclohex-3-enyl, and the like.

The term "cycloalkylcarbonyl" refers to a -C(O)-cycloalkyl group, wherein cycloalkyl is as defined above. Examples of cycloalkylcarbonyl are cyclopropylcarbonyl, cyclobutylcarbonyl, cyclopentylcarbonyl, cyclohexylcarbonyl and the like.

The term "dialkylamino" refers to a -N(alkyl)₂ group, wherein alkyl is as defined above and wherein each alkyl may be the same or different. Examples of dialkylamino groups are dimethylamino, diethylamino, ethylmethylamino and the like.

The term "dialkylaminoalkyleneoxy" refers to a -O-alkylene-N(alkyl)₂ group, wherein alkyl and alkylene are as defined above and wherein each alkyl may be the same or different. Examples of dialkylaminoalkyleneoxy groups are dimethylaminomethoxy, diethylaminomethoxy, ethylmethylaminomethoxy, dimethylaminoethoxy, diethylaminoethoxy, ethylmethylaminoethoxy and the like.

The term "dialkylaminocarbonyl" refers to a -C(O)N(alkyl)₂ group, wherein alkyl is as defined above and wherein each alkyl may be the same or different. Examples of dialkylaminocarbonyl groups are dimethylaminocarbonyl, diethylaminocarbonyl, ethylmethylaminocarbonyl and the like.

The term "dialkylaminocarbonyloxy" refers to a -O-C(O)N(alkyl)₂ group, wherein alkyl is as defined above and wherein each alkyl may be the same or different. Examples of dialkylaminocarbonyloxy groups are dimethylaminocarbonyloxy, diethylaminocarbonyloxy, ethylmethylaminocarbonyloxy and the like.

The terms "halogen" and "halo" refer to any one of F, Cl, Br and I.

The term "heteroaryl" means a monocyclic, fused bicyclic, or fused tricyclic, group of 5 to 14 ring atoms, preferably from 5 to 6 ring atoms, containing one or more, specifically one, two, three, or four ring heteroatoms independently selected from O, N and S, and the remaining ring atoms being carbon. In the case of monocyclic heteroaryl groups the ring will be aromatic and in the case of fused bicyclic or fused tricyclic groups at least one of the fused rings will be aromatic. One or two ring atoms of any nonaromatic rings may be replaced by a -C(O)- or -C(S)-, group. Examples of heteroaryl groups are pyridine, furane, thiophene, quinoline, tetrahidroquinoline and the like

The term "heterocyclyl" means a saturated or partially unsaturated (but not aromatic) monocyclic group of 3 to 8 ring atoms, preferably 5 to 7 ring atoms, or a saturated or partially unsaturated (but not aromatic) fused bicyclic group of 5 to 12 ring atoms in which one or more, specifically one, two, three of four ring heteroatoms are independently selected from N, O, and S. The heteroatoms may be substituted forming -S(O)n- (n is 0, 1, or 2), -. One or two ring atoms may be replaced by a-C(O)-, -C(S)-, or -C(=NH)- group. Fused bicyclic radical includes bridged ring systems. Examples of heterocyclyl are morpholinyl, piperidinyl, piperazinyl, and pyrrolidinyl.

The term "hydroxyalkyleneoxy" refers to a -O-alkylene-OH group, wherein alkylene is as defined above. Examples of hydroxyalkyleneoxy are hydroxymethoxy, 2-hydroxyethoxy, 3-hydroxypropoxy and the like.

The term "hydroxyl" or "hydroxy" refers to a -OH group.

As used herein, the term "pharmaceutically acceptable salt" embraces salts with a pharmaceutically acceptable acid or base, which are synthesized from the parent compound which contains an acidic moiety by addition of a pharmaceutically acceptable base, or which are synthesized from the parent compound which contains a basic moiety by addition of a pharmaceutically acceptable acid. Pharmaceutically acceptable acids include both inorganic acids, for example, hydrochloric, sulfuric, phosphoric, diphosphoric, hydrobromic, hydroiodic, and nitric acid, and organic acids, for example, citric, fumaric, maleic, malic, mandelic, ascorbic, oxalic, succinic, tartaric, benzoic, acetic, methanesulfonic (mesylate), ethanesulfonic, benzenesulfonic (besylate), or p-toluenesulfonic (tosylate) acid. Pharmaceutically acceptable bases include alkali metal (e.g., sodium or potassium) and alkali earth metal (e.g., calcium or magnesium) hydroxides and organic bases, such as alkyl amines, arylalkyl amines, and heterocyclic amines. For instance, pharmaceutically acceptable salts of compounds provided herein are synthesized from the parent compound which contains a basic or an acid moiety by conventional chemical methods. Generally, such salts are, for example, prepared by reacting the free base or free acid forms of these compounds with a stoichiometric amount of the appropriate acid or base, respectively, in water or in an organic solvent or in a mixture of the two. Preferably, the pharmaceutically acceptable salt is the mesylate.

As used herein, the term "stereoisomer" makes reference to compounds made up of the same atoms bonded by the same sequence of bonds but having different three-dimensional structures which are not interchangeable, depending on the presence of chiral centers, such as enantiomers, or diastereomers, or depending on the presence of multiple bonds, such as *Z* and *E* isomers. The single isomers, enantiomers or diastereoisomers and mixtures thereof fall within the scope of the present invention. If mixtures of stereoisomers are obtained, these isomers may be separated by conventional techniques such as preparative chromatography. If there are chiral centers the compounds may be prepared in racemic form, or individual enantiomers may be prepared either by enantiospecific synthesis or by resolution.

As used herein, the term "tautomer" makes reference to constitutional isomers of the compounds which differ in the position of a proton and a single bond adjacent to a double bond (as shown below), such as keto-enol tautomers, amide-imide tautomers, amine-imine tautomers, enamine-imine, lactam-lactim tautomer.

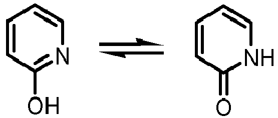

In particular, the following tautomers are encompassed by the present invention:

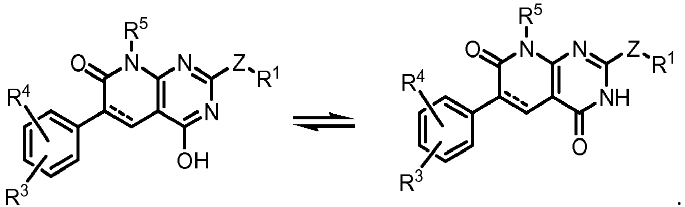

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The terms "treating" and "treatment", as used herein, mean reversing, alleviating, inhibiting the progress of infections caused by viruses of the *Flaviviridae* family, such as hepatitis C, Dengue fever, Japanese encephalitis, Kyasanur Forest disease, Murray Valley encephalitis, St. Louis encephalitis, Tick-borne encephalitis, West Nile encephalitis and yellow fever, in particular hepatitis C, or one or more symptoms associated with said diseases.

The terms "preventing" and "prevention", as used herein, means inhibiting the onset of infections caused by viruses of the *Flaviviridae* family, such as hepatitis C, Dengue fever, Japanese encephalitis, Kyasanur Forest disease, Murray Valley encephalitis, St. Louis encephalitis, Tick-borne encephalitis, West Nile encephalitis and yellow fever, in particular hepatitis C, or one or more symptoms associated with said diseases.

### Compounds of formula (I) and their pharmacological activity

The compounds of formula (I) of the present invention inhibit the replication of viruses of the *Flaviviridae* family, in particular of HCV.

Thus, in the first aspect, the present invention provides a compound of formula (I) as defined above, or a stereoisomer, tautomer or pharmaceutically acceptable salt thereof, for use in the treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever, preferably hepatitis C.

The present invention also relates to the use of a compound of formula (I) as defined above, or a stereoisomer, tautomer or pharmaceutically acceptable salt thereof, for manufacturing a medicament for the treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever, preferably hepatitis C.

The invention also relates to a method of treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever, preferably hepatitis C, in a subject in need thereof of, which comprises the administration of a therapeutically effective amount of a compound of formula (I) as defined above, or a stereoisomer, tautomer or pharmaceutically acceptable salt thereof.

In a preferred embodiment, the present invention provides a compound of formula (I) for use as defined above, wherein the bond represented by the dotted line is present, i.e. a compound of formula (III): 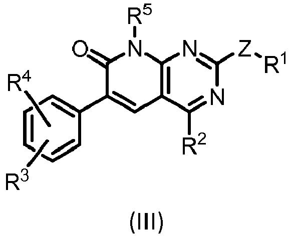 wherein R¹-R⁵ and Z are as defined for the compounds of formula (I).

In another preferred embodiment, the present invention provides a compound of formula (I) for use as defined above, wherein Z is NH.

In a particular embodiment, the present invention provides a compound of formula (I) for use as defined above, wherein R¹ is selected from H, benzyl, and phenyl optionally substituted with one, two or three substituents independently selected from the group consisting of C₁-C₃ alkoxy, F, Cl, Br, SO₂NH₂ and piperazinyl optionally substituted with C₁-C₃alkyl.

In a preferred embodiment, the present invention provides a compound of formula (I) for use as defined above, wherein R¹ is phenyl optionally substituted with one, two or three substituents independently selected from the group consisting of C₁-C₃ alkoxy, F, Cl, Br, and piperazinyl optionally substituted with C₁-C₃ alkyl; more preferably wherein R¹ is phenyl optionally substituted with one substituent selected from the group consisting of C₁-C₃ alkoxy, F, Cl, Br, and piperazinyl optionally substituted with C₁-C₃ alkyl; even more preferably wherein R¹ is phenyl optionally substituted with one substituent in *para* position with respect to the Z moiety selected from the group consisting of C₁-C₃ alkoxy, F, Cl, Br, and piperazinyl optionally substituted with C₁-C₃ alkyl; still more preferably wherein R¹ is phenyl optionally substituted with one substituent selected from the group consisting of methoxy, Br, and piperazinyl substituted with methyl; the most preferred, wherein R¹ is phenyl optionally substituted with one substituent in *para* position with respect to the Z moiety selected from the group consisting of methoxy, Br, and piperazinyl substituted with methyl.

In a particular embodiment, the present invention provides a compound of formula (I) for use as defined above, wherein R² is selected from the group consisting of H, -NR⁶R⁷, and -OR⁸, where R⁶, R⁷ and R⁸ are independently selected from the group consisting of H, C₁-C₆ alkyl and C₁-C₃ alkyl-O-C₁-C₃ alkylene, and benzotriazolyl, or R⁶ and R⁷ together with the nitrogen atom to which they are attached form a heterocyclyl selected form the group consisting of pyrrolidinyl, morpholinyl, piperidinyl, and piperazinyl, said heterocyclyl being optionally substituted with C₁-C₃ alkyl; preferably at least one of R⁶ and R⁷ is H.

In another particular embodiment, the present invention provides a compound of formula (I) for use as defined above, wherein R² is selected from the group consisting of H, -NR⁶R⁷, and -OR⁸, where R⁶ is H, and R⁷ and R⁸ are independently selected from the group consisting of H, C₁-C₆ alkyl and C₁-C₃ alkyl-O-C₁-C₃ alkylene, and benzotriazolyl, or R⁶ and R⁷ together with the nitrogen atom to which they are attached form a pyrrolidinyl; preferably at least one of R⁶ and R⁷ is H.

In another particular embodiment, the present invention provides a compound of formula (I) for use as defined above, wherein R² is selected from the group consisting of H, OH and NH₂, preferably OH and NH₂.

In a preferred embodiment, the present invention provides a compound of formula (I) for use as defined above, wherein R² is selected from the group consisting of OH and NH₂.

In a particular embodiment, the present invention provides a compound of formula (I) for use as defined above, wherein R³ and R⁴ are independently selected from the group consisting of H, F, Cl, Br, SO₂NH₂ and CF₃, or R³ and R⁴ together with the phenyl ring to which they are attached form a naphthyl; preferably wherein R³ and R⁴ are independently selected from the group consisting of H, F, Cl, Br and CF₃.

In a preferred embodiment, the present invention provides a compound of formula (I) for use as defined above, wherein R³ and R⁴ are independently selected from the group consisting of H, F, Cl, and Br, or R³ and R⁴ together with the phenyl ring to which they are attached form a naphthyl; preferably wherein R³ and R⁴ are independently selected from the group consisting of H, F, Cl, and Br.

In a particular embodiment, the present invention provides a compound of formula (I) for use as defined above, wherein R³ and R⁴ are simultaneously H, F, Cl, or Br, or R³ is H and R⁴ is F; preferably wherein R³ and R⁴ are in the *ortho* position with respect to the carbon atom bonded to the pyrido[2,3-d]pyrimidine-7(8H)-one ring system when said substituents are simultaneously H, F, Cl, or Br, or R⁴ is F and in the *para* position with respect to the carbon atom bonded to the pyrido[2,3-d]pyrimidine-7(8H)-one ring system and R³ is H.

In a particular embodiment, the invention provides a compound of formula (I) for use as defined above, wherein R⁵ is selected from the group consisting of H, C₁-C₆ alkyl optionally substituted one substituent selected from the group consisting of hydroxyl and C₁-C₆ alkoxy.

In a preferred embodiment, the invention provides a compound of formula (I) for use as defined above, wherein R⁵ is selected from the group consisting of H, C₁-C₃ alkyl optionally substituted one substituent selected from the group consisting of hydroxyl and C₁-C₃ alkoxy; preferably, wherein R⁵ is selected form the group consisting of H, methyl, ethyl, propyl, isopropyl, each of them being optionally substituted with methoxy or hydroxyl.

In a particular embodiment, the invention provides a compound of formula (I) for use as defined above, with the proviso that when the bond represented by the dotted line is absent and R² is OH, then R⁵ is not C₁-C₆ alkyl.

In a preferred embodiment, the present invention provides a compound of formula (I) for use as defined above, which is selected from the group consisting of:
- 6-(4-fluorophenyl)-2-(phenylamino)pyrido[2,3-d]pyrimidine-4,7(3H,8H)-dione,
- 4-amino-6-(2,6-difluorophenyl)-8-methyl-2-(phenylamino)pyrido[2,3-d]pyrimidine-7(8H)-one,
- 4-amino-6-(2,6-dichlorophenyl)-8-isobutyl-2-(phenylamino)pyrido[2,3-d] pyrimidine-7(8H)-one,
- 4-amino-2-((4-bromophenyl)amino)-6-(2,6-dichlorophenyl)-8-methylpyrido[2,3-d]pyrimidine-7(8H)-one,
- 6-(2,6-dichlorophenyl)-8-methyl-2-(phenylamino)pyrido[2,3-d]pyrimidine-7(8H)-one,
- 4-amino-6-(2,6-dichlorophenyl)-8-methyl-2-(phenylamino)pyrido[2,3-d] pyrimidine-7(8H)-one,
- 4-amino-6-(2,6-bromophenyl)-8-methyl-2-(phenylamino)pyrido[2,3-d] pyrimidine-7(8H)-one,
- 4-amino-6-phenyl-2-(phenylamino)pyrido[2,3-d]pyrimidine-7(8H)-one,
- 4-amino-8-methyl-6-phenyl-2-(phenylamino)pyrido[2,3-d]pyrimidine-7(8H)-one,
- 4-amino-6-(2,6-dichlorophenyl)-8-methyl-2-((4-(methylpiperazin-1-yl)phenyl) aminopyrido[2,3-d]pyrimidine-7(8H)-one,
- 4-amino-6-(2,6-dichlorophenyl)-8-(2-methoxyethyl)-2-(phenylamino)pyrido [2,3-d]pyrimidine-7(8H)-one,
- 4-((1H-benzo[d][1,2,3]triazol-1-yl)oxy)-6-(4-fluorophenyl)-2-(phenylamino)-3,4,5,6-tetrahydropyrido[2,3-d]pyrimidine-7(8H)-one,
- 4-((1H-benzo[d][1,2,3]triazol-1-yl)oxy)-6-(2,4-dichlorophenyl)-2-(phenylamino)-5,6-dihydropyrido[2,3-d]pyrimidine-7(8H)-one,
- 4-amino-6-(2,6-dichlorophenyl)-2-(phenylamino)-8-propylpyrido[2,3-d] pyrimidine-7(8H)-one,
- 2-amino-6-(4-fluorophenyl)-4-((2-propoxyethyl)amino)pyrido[2,3-d]pyrimidine-7(8H)-one,
- 4-amino-6-(2,6-dichlorophenyl)-8-(2-hydroxyethyl)-2-(phenylamino)pyrido[2,3-d]pyrimidine-7(8H)-one,
- 4-amino-6-(2-fluoro-6-(trifluoromethyl)phenyl)-8-methyl-2-(phenylamino) pyrido[2,3-d]pyrimidine-7(8H)-one,
- 2-amino-6-(2,6-dichlorophenyl)-4-(pentylamino)pyrido[2,3-d]pyrimidine-7(8H)-one,
- 6-(4-fluorophenyl)-2-phenoxy-4-((2-propoxyethyl)amino)pyrido[2,3-d] pyrimidine-7(8H)-one,
- 2-amino-6-(4-fluorophenyl)-8-methyl-4-((2-propoxyethyl)amino)pyrido[2,3-d] pyrimidine-7(8H)-one,
- 6-(2,6-dichlorophenyl)-8-methyl-2-(phenylamino)-4-((2-propoxyethyl)amino) pyrido[2,3-d]pyrimidine-7(8H)-one,
- 4-amino-6-(2,6-dichlorophenyl)-8-ethyl-2-(phenylamino)pyrido[2,3-d] pyrimidine-7(8H)-one,
- 4-amino-6-(2,6-dibromophenyl)-2-(phenylamino)pyrido[2,3-d]pyrimidine-7(8H)-one,
- 2-(benzylamino)-6-(2,6-dichlorophenyl)-5,6-dihydropyrido[2,3-d]pyrimidine-4,7(3H,8H)-dione,
- 2-amino-6-(2,6-dichlorophenyl)-8-methyl-4-(2-propoxyethoxy)pyrido[2,3-d] pyrimidine-7(8H)-one,
- 2-amino-6-(4-fluorophenyl)-8-methyl-4-(pentylamino)pyrido[2,3-d]pyrimidine-7(8H)-one,
- 4-amino-8-butyl-6-(2,6-dichlorophenyl)-2-(phenylamino)pyrido[2,3-d] pyrimidine-7(8H)-one,
- 6-(2,6-dichlorophenyl)-2-(phenylamino)-4-((2-propoxyethyl)amino)-5,6-dihydropyrido[2,3-d]pyrimidine-7(8H)-one,
- 4-amino-6-(2,6-dichlorophenyl)-8-methylpyrido[2,3-d]pyrimidine-7(8H)-one,
- 2-amino-6-(2,6-dichlorophenyl)-4-methoxy-8-methyl-5,6-dihydropyrido[2,3-d] pyrimidine-7(8H)-one,
- 4-amino-6-(2,6-difluorophenyl)-2-(phenylamino)pyrido[2,3-d]pyrimidine-7(8H)-one,
- 4-((1H-benzo[d][1,2,3]triazol-1-yl)oxy)-6-(4-fluorophenyl)-2-phenoxy-3,4,5,6-tetrahydropyrido[2,3-d]pyrimidine-7(8H)-one,
- 2-amino-6-(4-fluorophenyl)-8-methyl-4-(pentylamino)pyrido[2,3-d]pyrimidine-7(8H)-one,
- 2-amino-6-(2,6-dichlorophenyl)-4-(2-methoxyethoxy)-5,6-dihydropyrido[2,3-d] pyrimidine-7(8H)-one,
- 6-(2,6-difluorophenyl)-2-(phenylamino)-5,6-dihydropyrido[2,3-d]pyrimidine-4,7(3H,8H)-dione,
- 6-phenyl-2-(phenylamino)-5,6-dihydropyrido[2,3-d]pyrimidine-4,7(3H,8H)-dione,
- 6-phenyl-2-(phenylamino)- pyrido[2,3-d]pyrimidine-4,7(3H,8H)-dione, and
- 6-naphthalen-1-yl-2-(phenylamino)-5,6-dihydropyrido[2,3-d]pyrimidine-4,7(3H,8H)-dione, and
- 6-(4-fluorophenyl)-2-((4-methoxyphenyl)amino)pyrido[2,3-d]pyrimidine-4,7(3H,8H)-dione,
or a stereoisomer, tautomer or pharmaceutically acceptable salt thereof.

### Compounds of formula (II)

In the second aspect, the present invention provides a compound of formula (II) as defined above, or a stereoisomer, tautomer or pharmaceutically acceptable salt thereof, with the proviso that when R³ and R⁴ are both Cl in *ortho* position with respect to the carbon atom bonded to the pyrido[2,3-d]pyrimidine-7(8H)-one ring system, Z is NH, and R¹ is an optionally substituted phenyl, then R² is not NH₂.

In a particular embodiment, the present invention provides a compound of formula (II) as defined above, with the additional proviso that when Z is NH and R¹ is H, then R² is not NH₂.

In a particular embodiment, the present invention provides a compound of formula (II) as defined above, wherein R¹ is selected from H, benzyl, and phenyl optionally substituted with one, two or three substituents independently selected from the group consisting of F, Cl, Br, SO₂NH₂ and piperazinyl optionally substituted with C₁-C₃alkyl.

In a preferred embodiment, the present invention provides a compound of formula (II) as defined above, wherein R¹ is phenyl optionally substituted with one, two or three substituents independently selected from the group consisting of F, Cl, Br, and piperazinyl optionally substituted with C₁-C₃ alkyl; more preferably wherein R¹ is phenyl optionally substituted with one substituent selected from the group consisting of F, Cl, Br, and piperazinyl optionally substituted with C₁-C₃ alkyl; even more preferably wherein R¹ is phenyl optionally substituted with one substituent in *para* position with respect to the X moiety selected from the group consisting of F, Cl, Br, and piperazinyl optionally substituted with C₁-C₃ alkyl; still more preferably wherein R¹ is phenyl optionally substituted with one substituent selected from the group consisting of Br and piperazinyl substituted with methyl; the most preferred, wherein R¹ is phenyl optionally substituted with one substituent in *para* position with respect to the X moiety selected from the group consisting of Br and piperazinyl substituted with methyl.

In a particular embodiment, the present invention provides a compound of formula (II) as defined above, wherein R² is selected from the group consisting of-NR⁶R⁷, and -OR⁸, where R⁶, R⁷ and R⁸ are independently selected from the group consisting of H, C₁-C₆ alkyl and C₁-C₃ alkyl-O-C₁-C₃ alkylene, and benzotriazolyl, or R⁶ and R⁷ together with the nitrogen atom to which they are attached form a heterocyclyl selected form the group consisting of pyrrolidinyl, morpholinyl, piperidinyl, and pipeperazinyl, said heterocyclyl being optionally substituted with C₁-C₃ alkyl; preferably at least one of R⁶ and R⁷ is H.

In another particular embodiment, the present invention provides a compound of formula (II) as defined above, wherein R² is selected from the group consisting of - NR⁶R⁷, and -OR⁸, where R⁶ is H, and R⁷ and R⁸ are independently selected from the group consisting of H, C₁-C₆ alkyl and C₁-C₃ alkyl-O-C₁-C₃ alkylene, and benzotriazolyl, or R⁶ and R⁷ together with the nitrogen atom to which they are attached form a pyrrolidinyl; preferably at least one of R⁶ and R⁷ is H.

In a preferred embodiment, the present invention provides a compound of formula (II) as defined above, wherein R² is selected from the group consisting of OH and NH₂.

In another preferred embodiment the present invention provides a compound of formula (II) as defined above, wherein Z is NH.

In a particular embodiment, the present invention provides a compound of formula (II) as defined above, wherein R³ and R⁴ are independently selected from the group consisting of H, F, Cl, Br, SO₂NH₂ and CF₃, or R³ and R⁴ together with the phenyl ring to which they are attached form a naphthyl; preferably wherein R³ and R⁴ are independently selected from the group consisting of H, F, Cl, Br and CF₃.

In a particular embodiment, the present invention provides a compound of formula (II) as defined above, wherein R³ and R⁴ are independently selected from the group consisting of H, F, Cl, and Br, or R³ and R⁴ together with the phenyl ring to which they are attached form a naphthyl; preferably wherein R³ and R⁴ are independently selected from the group consisting of H, F, Cl, and Br.

In a particular embodiment, the present invention provides a compound of formula (II) as defined above, wherein R³ and R⁴ are simultaneously H, F, Cl, or Br, or R³ is H and R⁴ is F; preferably wherein R³ and R⁴ are in the *ortho* position with respect to the attachment point of the phenyl ring to the pyrido[2,3-d]pyrimidine-7(8H)-one core when said substituents are simultaneously H, F, Cl, or Br, or R⁴ is F and in the *para* position with respect to the attachment point of the phenyl ring to the pyrido[2,3-d]pyrimidine-7(8H)-one core and R³ is H.

In another particular embodiment, the invention provides a compound of formula (I) for use as defined above, wherein R⁵ is selected from the group consisting of H, C₁-C₆ alkyl optionally substituted one substituent selected from the group consisting of hydroxyl and C₁-C₆ alkoxy.

In another particular embodiment, the invention provides a compound of formula (II) as defined above, wherein R⁵ is selected from the group consisting of H, C₁-C₃ alkyl optionally substituted one substituent selected from the group consisting of hydroxyl and C₁-C₃ alkoxy; preferably, wherein R⁵ is selected form the group consisting of H, methyl, ethyl, propyl, isopropyl, each of them being optionally substituted with methoxy or hydroxyl.

In another particular embodiment, the invention provides a compound of formula (II) as defined above, wherein R⁵ is selected from the group consisting of C₁-C₃ alkyl optionally substituted one substituent selected from the group consisting of hydroxyl and C₁-C₃ alkoxy; preferably, wherein R⁵ is selected form the group consisting of methyl, ethyl, propyl, isopropyl, each of them being optionally substituted with methoxy or hydroxyl.

In a preferred embodiment, the present invention provides a compound of formula (II) which is selected from the group consisting of:
- 6-(4-fluorophenyl)-2-(phenylamino)pyrido[2,3-d]pyrimidine-4,7(3H,8H)-dione,
- 4-amino-6-(2,6-difluorophenyl)-8-methyl-2-(phenylamino)pyrido[2,3-d]pyrimidine-7(8H)-one,
- 4-amino-6-(2,6-bromophenyl)-8-methyl-2-(phenylamino)pyrido[2,3-d] pyrimidine-7(8H)-one,
- 4-amino-6-phenyl-2-(phenylamino)pyrido[2,3-d]pyrimidine-7(8H)-one,
- 4-amino-8-methyl-6-phenyl-2-(phenylamino)pyrido[2,3-d]pyrimidine-7(8H)-one,
- 2-amino-6-(4-fluorophenyl)-4-((2-propoxyethyl)amino)pyrido[2,3-d]pyrimidine-7(8H)-one,
- 4-amino-6-(2-fluoro-6-(trifluoromethyl)phenyl)-8-methyl-2-(phenylamino) pyrido[2,3-d]pyrimidine-7(8H)-one,
- 2-amino-6-(2,6-dichlorophenyl)-4-(pentylamino)pyrido[2,3-d]pyrimidine-7(8H)-one,
- 6-(4-fluorophenyl)-2-phenoxy-4-((2-propoxyethyl)amino)pyrido[2,3-d] pyrimidine-7(8H)-one,
- 2-amino-6-(4-fluorophenyl)-8-methyl-4-((2-propoxyethyl)amino)pyrido[2,3-d] pyrimidine-7(8H)-one,
- 6-(2,6-dichlorophenyl)-8-methyl-2-(phenylamino)-4-((2-propoxyethyl)amino) pyrido[2,3-d]pyrimidine-7(8H)-one,
- 4-amino-6-(2,6-dibromophenyl)-2-(phenylamino)pyrido[2,3-d]pyrimidine-7(8H)-one,
- 2-amino-6-(2,6-dichlorophenyl)-8-methyl-4-(2-propoxyethoxy)pyrido[2,3-d] pyrimidine-7(8H)-one,
- 2-amino-6-(4-fluorophenyl)-8-methyl-4-(pentylamino)pyrido[2,3-d]pyrimidine-7(8H)-one,
- 4-amino-6-(2,6-difluorophenyl)-2-(phenylamino)pyrido[2,3-d]pyrimidine-7(8H)-one,
- 2-amino-6-(4-fluorophenyl)-8-methyl-4-(pentylamino)pyrido[2,3-d]pyrimidine-7(8H)-one,
- 6-phenyl-2-(phenylamino)- pyrido[2,3-d]pyrimidine-4,7(3H,8H)-dione, and
- 6-(4-fluorophenyl)-2-((4-methoxyphenyl)amino)pyrido[2,3-d]pyrimidine-4,7(3H,8H)-dione,
or a stereoisomer, tautomer or pharmaceutically acceptable salt thereof.

The compounds of formula (II) fall under the definition of the compounds of formula (I). As explained above, these compounds are HCV inhibitors.

Thus, in another aspect, the present invention provides a compound of formula (II) as defined above, or a stereoisomer, tautomer or pharmaceutically acceptable salt thereof, for use as a medicament.

The present invention also provides the use of a compound of formula (II) as defined above, or a stereoisomer, tautomer or pharmaceutically acceptable salt thereof, in the manufacture of a medicament.

The present invention provides a compound of formula (II) as defined above, or a stereoisomer, tautomer or pharmaceutically acceptable salt thereof, for use in the treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever, preferably hepatitis C.

The present invention also provides the use of a compound of formula (II) as defined above, or a stereoisomer, tautomer or pharmaceutically acceptable salt thereof, for manufacturing a medicament for the treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever, preferably hepatitis C.

The invention also relates to a method of treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever, preferably hepatitis C, in a subject in need thereof of, which comprises the administration of a therapeutically effective amount of a compound of formula (II) as defined above, or a stereoisomer, tautomer or pharmaceutically acceptable salt thereof.

### Pharmaceutical compositions/formulations and administration

In another aspect, the present invention provides a pharmaceutical composition comprising a compound of formula (II) as defined above, or a stereoisomer, tautomer or pharmaceutically acceptable salt thereof, and one or more pharmaceutically acceptable excipients.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

Compounds of the invention may be administered by the oral, sublingual, parenteral, subcutaneous, intramuscular, intravenous, transdermal, intranasal, intraocular, and/or rectal routes. The compounds may be administered alone or in combination with one or more other compounds of the invention or one or more other drugs.

### Synthesis of compounds of formula (I) and formula (II)

The compounds of formula (II) represent a particular subgroup of the compounds of formula (I) wherein the double bond represented by the dotted line is present and the substituent at position 4 (R²) of the pyrido[2,3-d]pyrimidines is not a hydrogen atom.

Scheme 1 shows the synthetic pathway for obtaining compounds of formula (I) and compounds of formula (II) wherein Z is NH and R¹ is H.

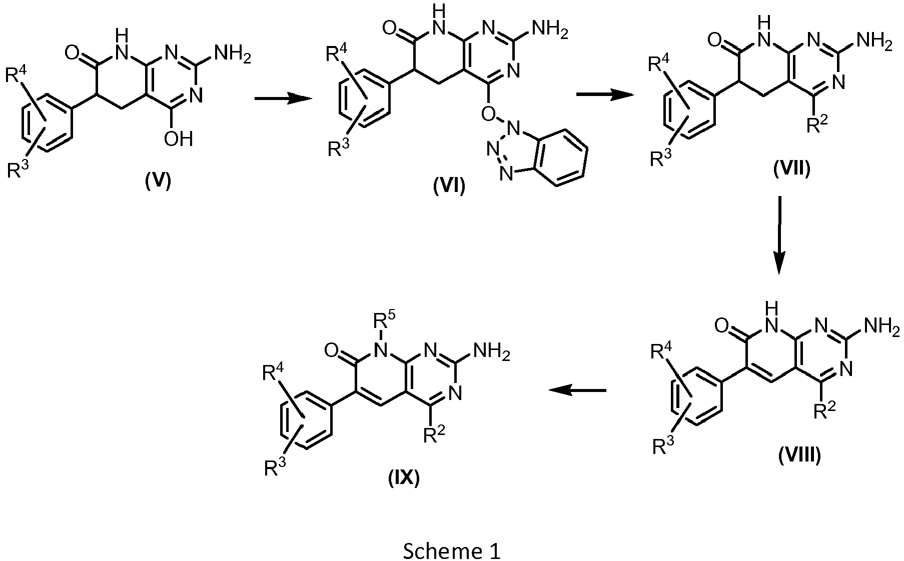

The starting material used for the synthesis compounds of formula (I) and formula (II) according to the pre sent invention pyrido[2,3-*d*]pyrimidine (**V**) wherein R³ and R⁴ are as defined for the compounds of formula (I), whose synthesis has been previously described [M. Camarasa et al., Mol. Diver. 2013, 17, 525-536].

Pyrido[2,3-*d*]pyrimidine (**V**) (which is a compound of formula (I) wherein R¹ is H, R² is OH, R³ and R⁴ are as defined above, R⁵ is H, Z is NH and the bond represented by the dotted line is absent) may be reacted with (benzotriazol-1-yloxy)tris(dimethylamino)phosphonium hexafluorophosphate (BOP) in a suitable solvent (e.g. acetonitrile, dioxane, tetrahydrofuran (THF), 1,2-dichloroethane (DCE), dichloromethane (DCM, CH₂Cl₂), N-methylpyrrolidone (NMP), dimethylformamide (DMF)) to give compound **(VI)** wherein R³ and R⁴ are as defined above (compound (**VI**) is a compound of formula (I) wherein R¹ is H, R² is O-benzotriazol, R³ and R⁴ are as defined above, R⁵ is H, Z is NH and the bond represented by the dotted line is absent).

The benzotriazolyl group in compound **(VI)** may be substituted by a nucleophile such as an amine (R⁶R⁷NH) in a suitable solvent (e.g. acetonitrile, DME, THF, DCM, NMP) the mixture was heated under microwave irradiation or by traditional heating from 90 to 140 ºC for 1 to 48 hours or an alcohol (R⁸OH) using the corresponding alcohol as a solvent in presence of caesium carbonate, the mixture was heated from 80 to 150 ºC for 5 to 24 hours leading to compounds **(VII),** wherein R³ and R⁴ are as defined above, and R² is -NR⁶R⁷, or -OR⁸ (compound **(VII)** is a compound of formula (I) wherein R¹ is H, R² is -NR⁶R⁷, or -OR⁸, R³ and R⁴ are as defined above, R⁵ is H, Z is NH and the bond represented by the dotted line is absent). In order to obtain compounds **(VII)** when R² is H the synthetic pathway described in X. Berzosa et. al. [J. Org. Chem. 2010, Vol. 75, No. 2, 487-490] may be followed. Compounds of formula **(VII)** wherein R² is NH₂ may also be obtained from compound **(VI)** by nucleophilic substitution with an amine (R⁶R⁷NH, wherein R⁶ and R⁷ are H) as described inRichard C. et. al. [Tetrahedron, 2009, vol 65, 8851-8857].

Compound **(VI)** may be dehydrogenated by reaction with 2,3-dichloro-5,6-dicyano-1,4-benzoquinone (DDQ) in 1,2-dichloroethane (DCE) or activated MnO₂ in acetic acid (AcOH) to give the corresponding dehydrogenated compound.

Compound **(VII)** may be dehydrogenated by reaction with NaH in dimethylsulfoxide (DMSO) or 2,3-dichloro-5,6-dicyano-1,4-benzoquinone (DDQ) in 1,2-dichloroethane (DCE) or activated MnO₂ in acetic acid (AcOH) to give compound **(VIII)** wherein R³ and R⁴ are as defined above, and R² is H, -NR⁶R⁷, or -OR⁸ (compound **(VIII)** is a compound of formula (I) wherein R¹ is H, R² is H, -NR⁶R⁷, or -OR⁸, R³ and R⁴ are as defined above, R⁵ is H, Z is NH and the bond represented by the dotted line is present; or a compound of formula (II) wherein R¹ is H, R² is -NR⁶R⁷ or -OR⁸, R³ and R⁴ are as defined above, R⁵ is H, and Z is NH).

Finally, compound **(VIII)** may be treated with NaH in DMSO and an halide R⁵-X, wherein X is a halogen and R⁵ is as defined above of the compounds of formula (I) except for H, for obtaining compound **(IX)** wherein R³-R⁵ are as defined above for the compounds of formula (I), and R² is H, -NR⁶R⁷, or -OR⁸ (compound **(IX)** is a compound of formula (I) wherein R¹ is H, R² is H, -NR⁶R⁷, or -OR⁸, R³ and R⁴ are as defined above, R⁵ is as defined for the compounds of formula (I) but different from hydrogen, Z is NH and the bond represented by the dotted line is present; or a compound of formula (II) wherein R¹ is H, R² is -NR⁶R⁷ or -OR⁸, R³ and R⁴ are as defined above, R⁵ is as defined for the compounds of formula (II) but different from hydrogen, and Z is NH).

Compound **(VII)** may also be treated with NaH in DMSO and an R⁵-X, wherein X is a halogen and R⁵ are as defined above of the compounds of formula (I) except for H, to give the corresponding compound of formula (I) wherein R⁵ is not hydrogen and the bond represented by the dotted line is not present.

Compound **(VI)** may also be dehydrogenated by reaction with DDQ in DCE or activated MnO₂ in AcOH, as explained with respect to Scheme 1, to give a compound of formula (I) wherein R¹, R³ and R⁴ are as defined for the compounds of formula (I), R² is O-benzotriazolyl, R⁵ is H, Z is NH, and the bond represented by the dotted line is present; or a compound of formula (II) wherein R¹, R³ and R⁴ are as defined for the compounds of formula (II), R² is O-benzotriazolyl, R⁵ is H and Z is NH. Compound (**V**) may also be dehydrogenated following the same procedure to give a compound of formula (I) wherein R¹, R³ and R⁴ are as defined for the compounds of formula (I), R² is OH, R⁵ is H, Z is NH and the bond represented by the dotted line is present; or a compound of formula (II) wherein R¹, R³ and R⁴ are as defined for the compounds of formula (II), R² is OH, R⁵ is H and Z is NH).

Scheme 2 shows the synthetic pathway for obtaining compounds of formula (I) and of formula (II) wherein Z is NH or O.

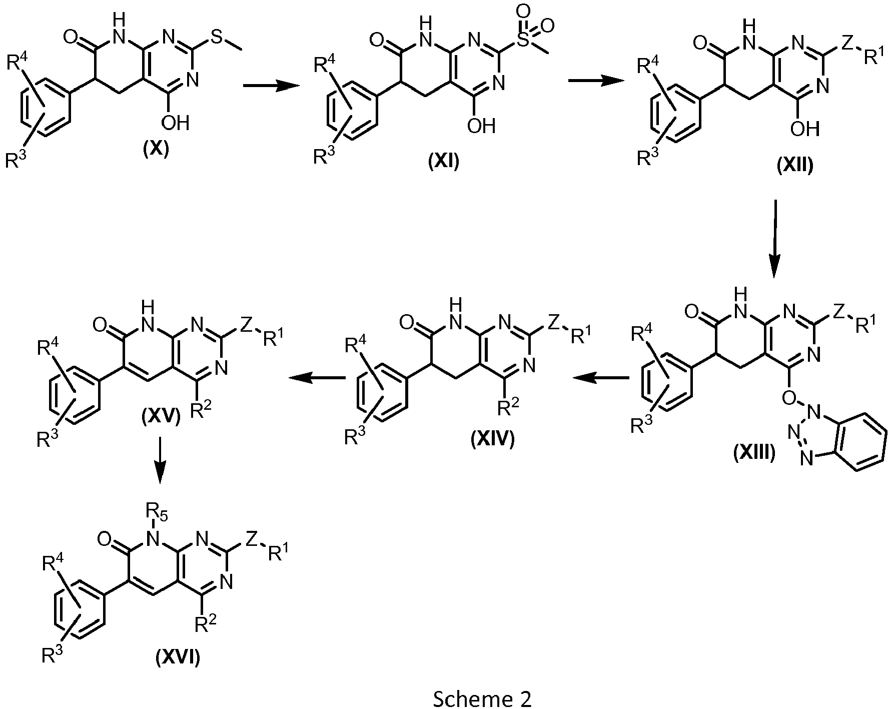

The starting material used for the synthesis compounds of formula (I) and formula (II) according to the present invention pyrido[2,3-*d*]pyrimidine (**X**), wherein R³ and R⁴ are as defined for the compounds of formula (I), whose synthesis has been previously described [M. Camarasa et al., Mol. Diver. 2013, 17, 525-536].

Then, the thiomethyl group in compound (**X**) may be oxidized into a sulfone group with *m*-CPBA in a suitable solvent (e.g. dimethylformamide), giving compound **(XI),** wherein R³ and R⁴ are as defined for the compounds of formula (I).

The next step is the reaction of compound **(XI)** with a suitable nucleophile such as an amine (R¹NH) in a suitable solvent (2-propanol, tert-butanol, acetonitrile, acetone, THF, toluene) and the mixture was heated under microwave irradiation or by traditional heating from 100 to 170 ºC for 1 to 12 hours or an alcohol (R¹OH) in a suitable solvent (2-propanol, tert-butanol, acetonitrile, acetone, THF, toluene) and the mixture was heated under microwave irradiation or by traditional heating from 100 to 170 ºC for 1 to 12 hours (when boiling point of solvent is upper than reaction temperature it has to work in a closed system), wherein R¹ is as defined above for the compounds of formula (I), in a suitable solvent (e.g. isopropanol) to afford compound **(XII)** wherein Z is NH or O, respectively, and R³ and R⁴ are as defined above (compound **(XII)** is a compound of formula (I) wherein R¹, R³ and R⁴ are as defined above, R² is OH, R⁵ is H, Z is NH or O, and the bond represented by the dotted line is absent).

Compound **(XII)** may be then reacted with BOP in a suitable solvent (e.g. acetonitrile) to give a compound **(XIII),** wherein R¹, R³, R⁴ and Z are as previously defined for compound **(XII)** (compound **(XIII)** is a compound of formula (I) wherein R¹, R³ and R⁴ are as defined above, R² is O-benzotriazolyl, R⁵ is H, Z is NH or O, and the bond represented by the dotted line is absent).

The benzotriazolyl group in compound **(XIII)** may be substituted by a nucleophile such as an amine (R⁶R⁷NH) in a suitable solvent (e.g. acetonitrile, DME, THF, DCM, NMP) the mixture was heated under microwave irradiation or by traditional heating from 90 to 140 ºC for 1 to 48 hours or an alcohol (R⁸OH) using the corresponding alcohol as a solvent in presence of sodium selenite, the mixture was heated from 80 to 150 ºC for 5 to 24 hours leading to compounds **(XIV),** wherein R¹, R³, R⁴ and Z are as defined above, and R² is -NR⁶R⁷, or -OR⁸ (which is a compound of formula (I) wherein R¹, R³, R⁴ and Z are as defined above, R² is -NR⁶R⁷, or -OR⁸, R⁵ is H and the bond represented by the dotted line is absent). In order to obtain compounds **(VII)** when R² is H the synthetic pathway described in X. Berzosa et. al. [J. Org. Chem. 2010, Vol. 75, No. 2, 487-490] has to be followed.

Compound **(XIV)** may be dehydrogenated by reaction with NaH in DMSO or DDQ in DCE or activated MnO₂ in AcOH to give compound **(XV)** wherein R¹-R⁴ and Z are as defined above (compound **(XV)** is a compound of formula (I) wherein R¹-R⁴ and Z are as defined above, R⁵ is H, and the bond represented by the dotted line is present; or a compound of formula (II) wherein R¹-R⁴ and Z are as defined for the compounds of formula (II), and R⁵ is H).

Finally, compound **(XV)** may be treated with NaH in DMSO and an R⁵-X, wherein X is a halogen and R⁵ is as defined above for compounds of formula (I) except for H, for obtaining compound **(XVI)** wherein R¹-R⁵ and Z are as defined above (compound **(XVI)** is a compound of formula (I) wherein R¹-R⁴ and Z are as defined above, R⁵ is as defined for the compounds of formula (I) but different from hydrogen, and the bond represented by the dotted line is present; or a compound of formula (II) wherein R¹-R⁴ and Z are as defined above, R⁵ is as defined for the compounds of formula (II) but different from hydrogen).

Compound **(XIV)** may also be treated with NaH in DMSO and an R⁵-X, wherein X and R⁵ are as defined above, to give the corresponding compound of formula (I) wherein R⁵ is not hydrogen and the bond represented by the dotted line is not present.

Compound **(XIV),** wherein R² is NH₂ may be dehydrogenated by reaction with NaH in DMSO or DDQ in DCE or activated MnO₂ in AcOH, as explained with respect to Scheme 1, to give a compound of formula (I) wherein R¹, R³, R⁴ and Z are defined for the compounds of formula (I), R² is NH₂, R⁵ is H, and the bond represented by the dotted line is present; or a compound of formula (II) wherein R¹, R³, R⁴ and Z are as defined for the compounds of formula (II), R² is NH₂ and R⁵ is H. Compound **(XIV)** and compound **(XV)** wherein R² is NH₂ may be treated with NaH in DMSO and an R⁵-X, wherein X and R⁵ are as defined above, to give the corresponding compound of formula (I) wherein R⁵ is not hydrogen and the bond represented by the dotted line is present or not, or a compound of formula (II) wherein R⁵ is not hydrogen.

Compound **(XIV)** may also be treated with sodium nitrite in a suitable solvent/reactant (e.g. acetic acid), for example by stirring a mixture of compound **(XIV)** and sodium nitrite at 25 ºC for 2 hours to give an intermediate compound wherein R² is an acyloxy (e.g. acetyloxy if acetic acid is used as the solvent/reactant), followed by the treatment of this intermediate with hydrochloric acid to give compound **(XVI),** wherein R¹, R³-R⁵ and Z are defined above and R² is OH (compound **(XVI)** is a compound of formula (I) wherein R¹, R³, R⁴ and Z are as defined above, R² is OH, R⁵ is as defined for the compounds of formula (I) but different from hydrogen, and the bond represented by the dotted line is present; or a compound of formula (II) wherein R¹, R³, R⁴ and Z are as defined above, R² is OH, R⁵ is as defined for the compounds of formula (II) but different from hydrogen).

Compound **(XII)** may also be dehydrogenated with NaH in DMSO or DDQ in DCE, as explained with respect to Scheme 1, to give a compound of formula (I) wherein R¹, R³, R⁴ and Z are as defined for the compounds of formula (I), R² is OH, R⁵ is H, and the bond represented by the dotted line is present; or a compound of formula (II) wherein R¹, R³, R⁴ and Z are as defined for the compounds of formula (II), R² is OH, and R⁵ is H).

Scheme 3 shows the synthetic pathway for obtaining compounds of formula (I) and of formula (II) wherein Z is NH, R¹ is phenyl and R² is NH₂.

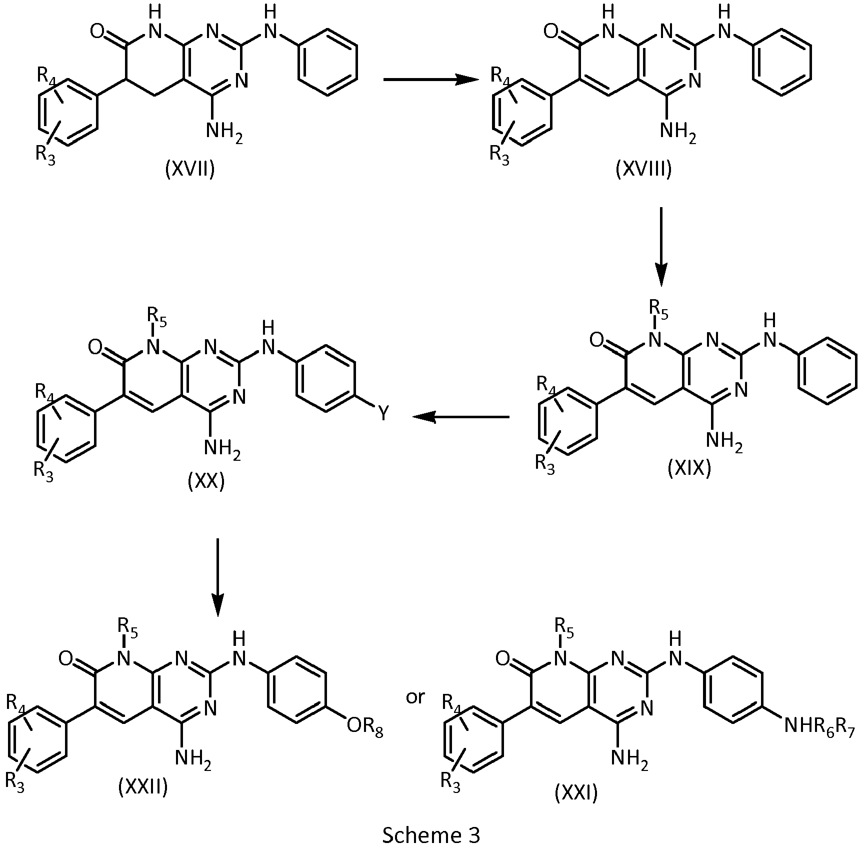

The starting material used for the synthesis compounds of formula (I) and formula (II) according to the present invention is pyrido[2,3-*d*]pyrimidine **(XVII),** wherein R³ and R⁴ are as defined for the compounds of formula (I), whose synthesis has been previously described [Galve et al., Mol. Diver. 2012, 16, 639-649].

Pyrido[2,3-d]pyrimidine **(XVII)** (which is a compound of formula (I) wherein R¹ is phenyl, R² is NH₂, R³ and R⁴ are as defined above, R⁵ is H, Z is NH and the bond represented by the dotted line is absent) may be dehydrogenated by reaction with NaH in dimethylsulfoxide (DMSO) or 2,3-dichloro-5,6-dicyano-1,4-benzoquinone (DDQ) in 1,2-dichloroethane (DCE) or activated MnO₂ in acetic acid (AcOH) to give compound **(XVIII)** wherein R³ and R⁴ are as defined above (compound **(XVIII)** is a compound of formula (I) wherein R¹ is Phenyl, R² is NH₂, R³ and R⁴ are as defined above, R⁵ is H, Z is NH and the bond represented by the dotted line is present; or a compound of formula (II) wherein R¹ is phenyl, R² is NH₂, R³ and R⁴ are as defined above, R⁵ is H, and Z is NH).

Compound **(XVIII)** may be treated with NaH in DMSO and an halide R⁵-X, wherein X is a halogen and R⁵ is as defined above of the compounds of formula (I) except for H, for obtaining compound **(XIX)** wherein R³-R⁵ are as defined above for the compounds of formula (I), and R2 is NH₂, (compound **(XIX)** is a compound of formula (I) wherein R¹ is phenyl, R² is NH₂, R³ and R⁴ are as defined above, R⁵ is as defined for the compounds of formula (I) but different from hydrogen, Z is NH and the bond represented by the dotted line is present; or a compound of formula (II) wherein R¹ is phenyl, R² is NH₂, R³ and R⁴ are as defined above, R⁵ is as defined for the compounds of formula (II) but different from hydrogen, and Z is NH).

Compound **(XVII)** may also be treated with NaH in DMSO and an R⁵-X, wherein X is a halogen and R⁵ are as defined above of the compounds of formula (I) except for H, to give the corresponding compound of formula (I) wherein R⁵ is not hydrogen and the bond represented by the dotted line is not present.

Compound **(XIX)** may be halogenated in the *para* position of the 2-phenylamino substituent by reaction with a halogenating agent as explained with respect to Scheme 3, to give a compound of formula (I) wherein R¹ is phenyl, R³ and R⁴ are as defined for the compounds of formula (I), R² is NH₂, R⁵ is not H, Z is NH, and the bond represented by the dotted line is present; or a compound of formula (II) wherein R¹ is phenyl, R³ and R⁴ are as defined for the compounds of formula (I), R² is NH₂, R⁵ is not H and Z is NH. Halogenating agents are known in the art and typically consist in the use of the corresponding *N*-halo succinimide or the corresponding Y₂, wherein Y and "halo" is selected from the group of I, Br, Cl, preferably Br. Preferably, the halogenating agent is a brominating agent, i.e. Y is Br. Preferably, the brominating agent is selected from the group consisting of Br₂ in acetic acid or N-bromosuccinimide in acetic acid, more preferably, Br₂ in acetic acid. Preferred reaction conditions are the use of between 0.8:1.5 and 1.5:0.8 molar amounts of the corresponding pyrido[2,3-*d*]pyrimidine **(XIX)** with respect to bromine in acetic acid, preferably 2M bromine in acetic acid solution, preferably between 0.9:1.1 and 1.1:0.9 molar amounts of the corresponding pyrido[2,3-*d*]pyrimidine **(XIX)** with respect to bromine in acetic acid, preferably 2M bromine in acetic acid solution, even more preferably equimolar amounts of the corresponding pyrido[2,3-*d*]pyrimidine **(XIX)** with respect to bromine in acetic acid, preferably 2M bromine in acetic acid solution. This reaction may be carried out in the presence of an organic solvent such as 1,4-dioxane or ,THF and is carried out at room temperature. Compound **(XVII)** and compound **(XVIII)** may also be halogenated in the *para* position of the 2-phenylamino substituent following the same procedure to give a compound of formula (I) wherein R¹ is phenyl, R³ and R⁴ are as defined for the compounds of formula (I), R² is NH₂, R⁵ is H, Z is NH and the bond represented by the dotted line is present; or a compound of formula (II) wherein R¹ is phenyl, R³ and R⁴ are as defined for the compounds of formula (II), R² is NH₂, R⁵ is H and Z is NH).

Finally, halogen atom Y in compound **(XX)** may be substituted by a nucleophile such as an amine (R⁶R⁷NH) or an alcohol (R⁸OH) by using Ullman reaction conditions. This step is carried out by reacting the compound of formula **(XX)** in the presence of a copper catalyst with a compound R⁶R⁷-NH to give compounds **(XXI)** wherein R³-R⁷ are as defined above, R² is NH₂ (compound **(XXI)** is a compound of formula (I) wherein R¹ is phenyl substituted in *para* position by -NR⁶R⁷, R² is NH₂, R³-R⁷ are as defined above, Z is NH and the bond represented by the dotted line is present; or a compound of formula (II) wherein R¹ is phenyl substituted in *para* position by -NR⁶R⁷, R² is NH₂, R³-R⁷ are as defined above, and Z is NH), or reacting the compound of formula **(XX)** with a compound R⁸-OH, wherein R⁸ to give a compound of formula **(XXII)** wherein R⁸ is defined above, R² is NH₂ (compound **(XXII)** is a compound of formula (I) wherein R¹ is phenyl substituted in *para* position by -OR⁸, R² is NH₂, R³-R⁷ are as defined above, Z is NH and the bond represented by the dotted line is present; or a compound of formula (II) wherein R¹ is phenyl substituted in *para* position by -OR⁸, R² is NH₂, R³-R⁷ are as defined above, and Z is NH). Preferably, the copper catalyst of is metallic copper, copper-bronze, Cu₂Br₂, Cu₂O, Cu₂I₂, preferably Cu₂I₂, and the reaction is performed in the presence of a base, such as K₂CO₃, Na₂CO₃, Cs₂CO₃, and the like, preferably K₂CO₃, and a ligand selected from the group consisting of L-proline, ethyleneglycol, N,N-dimethylcyclohexane-1,2-diamine, and N,N-diethylsalicylamide, or the like, preferably, L-proline [J. Kim and S. Chang, Chem. Commun. 2008, 3052-3054]. This step may be carried out in an organic solvent such as dimethylsulfoxide, dimethylformamide, preferably dimethylsulfoxide. The reaction is preferably performed with heating, such as between 60 ºC and 200 ºC, preferably between 60 ºC and 100 ºC. In a particular embodiment, the heating is achieved by means of microwave irradiation, but conventional heating means may also be employed.

Scheme 4 shows the synthetic pathway for obtaining compounds of formula (I) and of formula (II) wherein Z is NH R¹ is H and R² is NH₂.

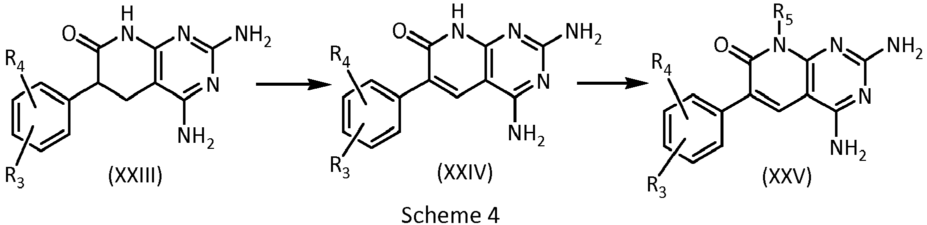

The starting material used for the synthesis compounds of formula (I) and formula (II) according to the present invention is pyrido[2,3-*d*]pyrimidine **(XXIII),** wherein R³ and R⁴ are as defined for the compounds of formula (I), whose synthesis has been previously described [Martínez-Teipel B et al., J Comb Chem 2005, 7, 436-448].

Pyrido[2,3-*d*]pyrimidine **(XXIII)** (which is a compound of formula (I) wherein R¹ is H, R² is NH₂, R³ and R⁴ are as defined above, R⁵ is H, Z is NH and the bond represented by the dotted line is absent) may be dehydrogenated by reaction with NaH in dimethylsulfoxide (DMSO) or 2,3-dichloro-5,6-dicyano-1,4-benzoquinone (DDQ) in 1,2-dichloroethane (DCE) or activated MnO₂ in AcOH to give compound **(XXIV)** wherein R³ and R⁴ are as defined above (compound **(XXIV)** is a compound of formula (I) wherein R¹ is H, R² is NH₂, R³ and R⁴ are as defined above, R⁵ is H, Z is NH and the bond represented by the dotted line is present; or a compound of formula (II) wherein R¹ is H, R² is NH₂, R³ and R⁴ are as defined above, R⁵ is H, and Z is NH).

Compound **(XXIV)** may be treated with NaH in DMSO and an halide R⁵-X, wherein X is a halogen and R⁵ is as defined above of the compounds of formula (I) except for H, for obtaining compound **(XXV)** wherein R³-R⁵ are as defined above for the compounds of formula (I), and R² is NH₂, (compound **(XXV)** is a compound of formula (I) wherein R¹ is H, R² is NH₂, R³ and R⁴ are as defined above, R⁵ is as defined for the compounds of formula (I) but different from hydrogen, Z is NH and the bond represented by the dotted line is present; or a compound of formula (II) wherein R¹ is H, R² is NH₂, R³ and R⁴ are as defined above, R⁵ is as defined for the compounds of formula (II) but different from hydrogen, and Z is NH).

Compound **(XXIII)** may also be treated with NaH in DMSO and an R⁵-X, wherein X is a halogen and R⁵ are as defined above of the compounds of formula (I) except for H, to give the corresponding compound of formula (I) wherein R⁵ is not hydrogen and the bond represented by the dotted line is not present.

The following examples represent specific embodiments of the present invention. They do not intend to limit in any way the scope of the invention defined in the present description.

The following abbreviations are employed herein:
- ACN: acetonitrile
- AcOH: acetic acid
- BOP: benzotriazol-1-yloxy)tris(dimethylamino)phosphonium hexafluoro phosphate
- *m*-CPBA: *m*-chloroperbenzoic acid
- DBU: 1,8-diazabicycloundec-7-ene
- DCE: dichloroethane
- DDQ: 2,3-dichloro-5,6-dicyanobenzoquinone
- DMF: N,N-dimethylformamide
- DMSO: dimethylsulfoxide
- EtOH: ethanol
- EtOEt or Et₂O: diethyl ether
- IPA: isopropanol
- MeOH: methanol
- NMR: nuclear magnetic resonance
- TFA: trifluoroacetic acid

### Examples

### Materials and methods

¹H and ¹³C NMR spectra were recorded on a Varian 400-MR spectrometer that was operating at a field strength of 400 and 100.6 MHz, respectively. Chemical shifts were reported in parts per million (d) and coupling constants (J) were in Hz by using, in the case of ¹H NMR spectroscopy, TMS as an internal standard, and in the case of ¹³C NMR spectroscopy the solvent at 39.5 ppm (DMSO-*d₆*) or at 29.84 ppm (acetone-*d₆*) or at 77.0 ppm (CDCl₃) as an internal reference. Standard and peak multiplicities are designed as follows: s, singlet; d, doublet; t, triplet; q, quartet; quint, quintuplet; hex, hexuplet; hept, heptuplet; br, broad signal.

Automatic flash chromatography was performed in an Isco Combiflash medium pressure liquid chromatograph with Redi*Sep*^{®} silica gel columns (35-70 µm) using a suitable mixture of solvents as eluent.

Microwave irradiation experiments were carried out in a Initiator™ (Biotage) microwave apparatus, operating at a frequency of 2.45 GHz with continuous irradiation power from 0 to 400 W. Reactions were carried out in 0.5, 2.5, 5, 20 mL glass tubes, sealed with aluminium/Teflon crimp tops, which can be exposed up to 250 ºC and 20 bar internal pressure. Temperature was measured with an IR sensor on the outer surface of the process vial. After the irradiation period, the reaction vessel was cooled rapidly to 50 ºC by air jet cooling.

### General procedure for the synthesis of 4-((1H-benzo[d][1,2,3]triazol-1-yl)oxy)-6-aryl-3,4,5,6-tetrahydropyrido[2,3-d]pyrimidine-7(8H)-ones

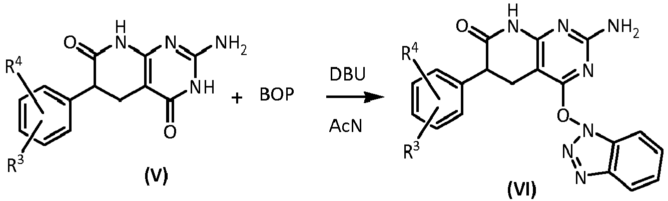

The starting material of pyrido[2,3-*d*]pyrimidine (V) (0.50 mmol) (synthesized as described in M. Camarasa et al., Mol. Diver. 2013, 17, 525-536) was suspended in 13 mL of anhydrous ACN. Then, BOP (0.75 mmol) and DBU (1.25 mmol) were added into the solution. The system was stirred at room temperature for 2 days under nitrogen atmosphere. After this period, 200 mL of water was added and the resulting precipitate was filtered, washed with water and dried *in vacuo* over phosphorus pentoxide to afford the corresponding 4-((1H-benzo[d][1,2,3]triazol-1-yl)oxy)-6-aryl-3,4,5,6-tetrahydropyrido[2,3-d]pyrimidine-7(8H)-one (VI) as a brownish solid.

### Example 1: Synthesis of 4-((1H-benzo[d][1,2,3]triazol-1-yl)oxy)-2-amino-6-(4-fluoro phenyl)-3,4,5,6-tetrahydropyrido[2,3-d]pyrimidine-7(8H)-one

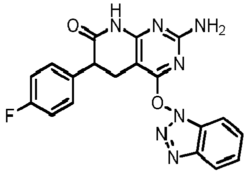

¹H NMR (400 MHz, DMSO-*d*₆): δ 10.95 (s, 1H), 8.13 (dt, *J* = 8.4, 0.9 Hz, 1H), 7.70 - 7.61 (m, 2H), 7.51 (ddd, *J* = 8.1, 6.4, 1.6 Hz), 7.39 (dd, *J* = 8.7, 5.6 Hz, 2H), 7.21 (t, *J* = 8.9 Hz, 1H), 6.59 (s, 2H, NH₂), 4.11 (dd, *J* = 10.7, 6.9 Hz, 1H), 3.29 - 3.12 (m, 2H).

### Example 2: Synthesis of 4-((1H-benzo[d][1,2,3]triazol-1-yl)oxy)-2-amino-6-(2,6-dichloro phenyl)-3,4,5,6-tetrahydropyrido[2,3-d]pyrimidine-7(8H)-one

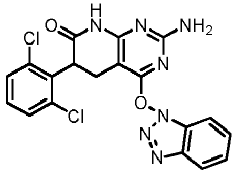

¹H NMR (400 MHz, DMSO-*d*₆): δ 11.00 (s, 1H), 8.13 (dt, *J* = 8.4, 0.9 Hz, 1H), 7.81 (dt, *J* = 8.4, 1.0 Hz, 1H), 7.68 - 7.61 (m, 1H), 7.60 - 7.46 (m, 3H), 7.39 (t, *J* = 8.1 Hz, 1H), 6.63 (s, 2H, NH₂), 4.91 (dd, *J* = 12.6, 9.3 Hz, 1H), 3.31 - 3.16 (m, 2H).

¹³C NMR (100.6 MHz, DMSO-*d*₆): 170.14, 166.17, 161.79, 160.48, 143.17, 135.79, 135.38, 135.23, 130.39, 130.20, 129.42, 128.94, 128.86, 125.58, 120.22, 110.05, 84.62, 43.20, 21.98.

### Example 3: Synthesis of 4-((1H-benzo[d][1,2,3]triazol-1-yl)oxy)-6-(4-fluorophenyl)-2-phenoxy-3,4,5,6-tetrahydropyrido[2,3-d]pyrimidine-7(8H)-one

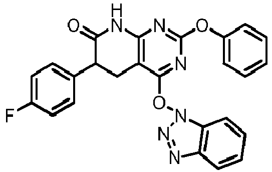

¹H NMR (400 MHz, DMSO-*d*₆): δ 11.50 (s, 1H), 8.07 (dt, *J* = 8.4, 0.9 Hz, 1H), 7.70-7.60 (m, 2H), 7.53 - 7.49 (m, 1H), 7.41 (dd, *J* = 8.7, 5.5 Hz, 2H), 7.22 (t, *J* = 8.9 Hz, 2H), 7.11 - 7.00 (m, 3H), 6.84 - 6.78 (m, 2H), 4.20 (dd, *J* = 11.5, 7.1 Hz, 1H), 3.44 - 3.34 (m, 2H).

### Example 4: 4-((1H-benzo[d][1,2,3]triazol-1-yl)oxy)-6-(4-fluorophenyl)-2-(phenylamino)-3,4,5,6-tetrahydropyrido[2,3-d]pyrimidine-7(8H)-one

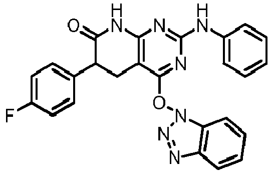

¹H NMR (400 MHz, DMSO-*d*₆): δ 11.19 (s, 1H), 9.46 (s, 1H), 8.23 (d, *J* = 8.5 Hz, 1H), 7.76 (dt, *J* = 8.4, 0.9 Hz, 1H), 7.69 - 7.64 (m, 1H), 7.56 (ddd, *J* = 8.2, 6.9, 1.1 Hz, 1H), 7.43 (dd, *J* = 8.7, 5.5 Hz, 2H), 7.23 (t, *J* = 8.9 Hz, 2H), 6.95 - 6.90 (m, 2H), 6.85 - 6.79 (m, 2H), 6.75 - 6.77 (m, 1H), 4.19 (dd, *J* = 11.0, 7.0 Hz, 1H), 3.41 - 3.29 (m, 2H).

### Example 5: Synthesis of 4-((1H-benzo[d][1,2,3]triazol-1-yl)oxy)-6-(2,6-dichlorophenyl)-2-(phenylamino)-5,6-dihydropyrido[2,3-d]pyrimidine-7(8H)-one

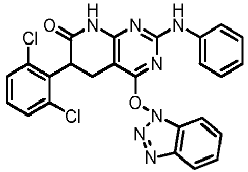

¹H NMR (400 MHz, DMSO-*d*₆) δ 11.24 (s, 1H), 9.47 (s, 1H), 8.22 (d, *J* = 8.4 Hz, 1H), 7.88 (d, *J* = 8.3 Hz, 1H), 7.66 (t, *J* = 7.1 Hz, 1H), 7.63 - 7.52 (m, 3H), 7.42 (t, *J* = 8.1 Hz, 1H), 6.97 (m, 2H), 6.82 (m, 2H), 6.78 - 6.72 (m, 1H), 5.01 (dd, *J* = 12.5, 9.3 Hz, 1H), 3.38 - 3.34 (m, 1H), 2.75 - 2.68 (m, 1H).

### Example 6: 4-((1H-benzo[d][1,2,3]triazol-1-yl)oxy)-6-(2,4-dichlorophenyl)-2-(phenylamino)-5,6-dihydropyrido[2,3-d]pyrimidine-7(8H)-one

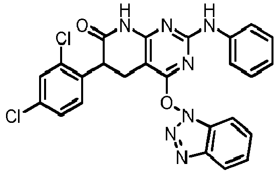

¹H NMR (400 MHz, DMSO-*d*₆) δ 11.27 (s, 1H), 9.48 (s, 1H), 8.22 (d,J=8.4 Hz, 1H), 7.81 (dt, *J* = 8.4, 0.9 Hz, 1H), 7.70 (d, *J* = 2.2 Hz, 1H), 7.66 (ddd, *J* = 8.3, 6.9, 0.9 Hz, 1H), 7.58 (d, *J* = 8.4 Hz, 1H), 7.56 (ddd, *J* = 8.1, 6.9, 1.0 Hz, 1H), 7.51 (dd, *J* = 8.4, 2.2 Hz, 1H), 7.03 - 6.78 (m, 4H), 6.78 - 6.72 (m, 1H), 4.55 (dd, *J* = 11.6, 9.0 Hz, 1H), 3.35 (d, *J* = 8.9 Hz, 2H).

### General procedure for the synthesis of 4-amino-substituted pyrido[2,3-d]pyrimidines with amine

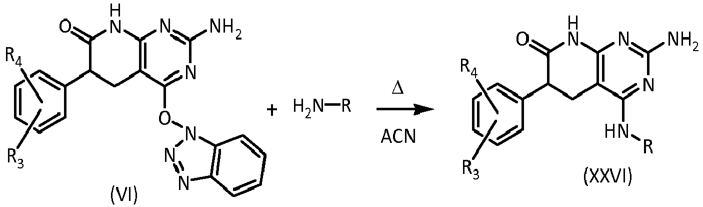

The corresponding 4-((1H-benzo[d][1,2,3]triazol-1-yl)oxy)-6-aryl-3,4,5,6-tetrahydropyrido[2,3-d]pyrimidine-7(8H)-one (VI) (0.42 mmol) was suspended in 20 mL of ACN, the correspondent amine (0.84 mmol) was added and the system was heated under microwave irradiation at 140 ºC for 5 hours. The resulting solution was cooled down, water (100 mL) was added and the solid was collected by filtration and washed with water, and EtOEt to afford the corresponding 4-amino-substituted pyrido[2,3-d]pyrimidine (XXVI).

### Example 7: Synthesis of 2-amino-6-(4-fluorophenyl)-4-((2-propoxyethyl)amino)-5,6-dihydropyrido[2,3-d]pyrimidine-7(8H)-one

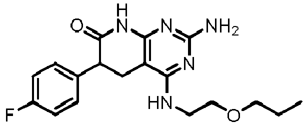

¹H NMR (400 MHz, CDCl₃): δ 11.99 (s, 1H), 7.26 - 7.20 (m, 2H), 7.04 (t, *J* = 8.7 Hz, 2H, 4.81 - 4.75 (m, 1H), 3.84 (dd, *J* = 10.2, 7.3 Hz, 1H), 3.64 - 3.61 (m, 2H), 3.58 - 3.52 (m, 2H), 3.39 (t, *J* = 6.7 Hz), 2.85 - 2.67 (m, 2H), 1.63 - 1.51 (m, 2H), 0.88 (t, *J* = 7.4 Hz, 3H).

### Example 8: Synthesis of 2-amino-6-(4-fluorophenyl)-4-(pentylamino)-5,6-dihydro pyrido[2,3-d]pyrimidine-7(8H)-one

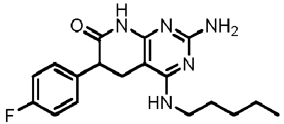

¹H NMR (400 MHz, acetone): δ 7.38 (m, 2H), 7.11 (t, *J* = 8.8 Hz, 2H), 3.94 (dd, *J* = 10.3, 7.2 Hz, 1H), 3.42 (dd, *J* = 12.9, 6.6 Hz, 2H), 2.87 (m, 2H), 1.67 - 1.51 (m, 2H), 1.41 -1.25 (m, 4H), 0.88 (t, *J* = 6.8 Hz, 3H).

### Example 9: Synthesis of 2-amino-6-(4-fluorophenyl)-4-(pyrrolidin-1-yl)-5,6-dihydro pyrido[2,3-d]pyrimidine-7(8H)-one

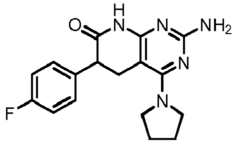

¹H NMR (400 MHz, DMSO-*d*₆): δ 10.31 (s, 1H), 7.30 - 7.24 (m, 2H), 7.14 (t, *J* = 8.9 Hz, 2H), 5.89 (s, 2H), 3.78 (dd, *J* = 10.4, 6.6 Hz, 1H), 3.50 - 3.41 (m, 4H), 3.16 - 3.02 (m, 2H), 1.85 - 1.67 (m, 4H).

### Example 10: Synthesis of 6-(4-fluorophenyl)-2-phenoxy-4-((2-propoxyethyl)amino)-5,6-dihydropyrido[2,3-d]pyrimidine-7(8H)-one

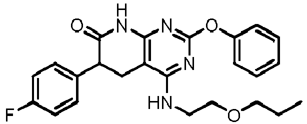

¹H NMR (400 MHz, DMSO-*d*₆): δ 8.01 (s, 1H), 7.35 (t, *J* = 7.7 Hz, 2H), 7.23 - 7.12 (m, 5H), 7.01 (t, *J* = 8.6 Hz, 2H), 5.14 (s, 1H), 3.83 (dd, *J* = 9.7, 7.2 Hz, 1H), 3.61 - 3.44 (m, 4H), 3.35 (t, *J* = 6.7 Hz, 2H), 2.94 - 2.68 (m, 2H), 1.54 (hex, *J* = 7.1 Hz, 2H), 0.87 (t, *J* = 7.4 Hz, 3H).

### Example 11: Synthesis of 6-(2,6-dichlorophenyl)-2-(phenylamino)-4-((2-propoxy ethyl)amino)-5,6-dihydropyrido[2,3-d]pyrimidine-7(8H)-one

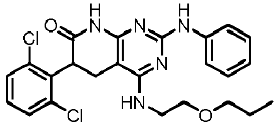

¹H NMR (400 MHz, DMSO-*d*₆) δ 10.31 (s, 1H), 8.82 (s, 1H), 7.83 (dd, *J* = 8.7, 1.0 Hz, 2H), 7.54 (m, 2H), 7.39 (t, *J* = 8.1 Hz, 1H), 7.19 (dd, *J* = 8.5, 7.4 Hz, 2H), 6.85 (t, *J* = 7.3 Hz, 1H), 6.60 (s, 1H), 4.68 (dd, *J* = 13.1, 8.9 Hz, 1H), 3.58 - 3.48 (m, 4H), 3.35 (t, *J* = 6.6 Hz, 2H), 2.99 - 2.90 (m, 1H), 2.77 (dd, *J* = 15.7, 13.2 Hz, 1H), 1.49 (hex, *J* = 7.3 Hz, 2H), 0.84 (t, *J* = 7.4 Hz, 3H).

¹³C NMR (100.6 MHz, DMSO-*d*₆) δ 169.2, 160.1, 158.1, 155.1, 141.4, 135.6, 135.2, 134.8, 129.8, 129.8, 128.3, 128.2, 120.3, 118.4, 84.6, 71.7, 68.6, 43.1, 40.3, 23.4, 22.5, 10.5.

### General procedure for the synthesis of 4-alkoxy-substituted pyrido[2,3-d]pyrimidines

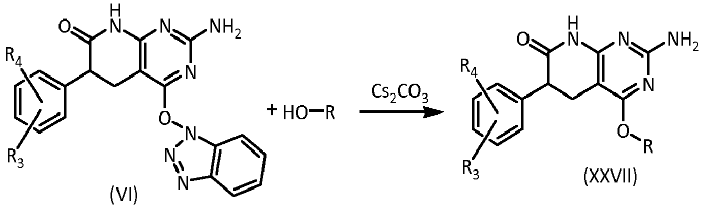

A mixture of the corresponding 4-((1H-benzo[d][1,2,3]triazol-1-yl)oxy)-6-aryl-3,4,5,6-tetrahydropyrido[2,3-d]pyrimidine-7(8H)-one (VI) (0.42 mmol), Cs₂CO₃ (0.54 mmol) and the corresponding alcohol (9 ml) was heated a reflux overnight. The resulting solution was cooled down, water (100 mL) was added and the reaction crude was neutralized with 2M HCl. The solid was collected by filtration and washed with water, and EtOEt to afford the corresponding 4-alkoxy-substituted pyrido[2,3-d]pyrimidine (XXVII).

### Example 12: Synthesis of 2-amino-6-(2,6-dichlorophenyl)-4-(2-propoxyethoxy)-5,6-dihydropyrido[2,3-d]pyrimidine-7(8H)-one

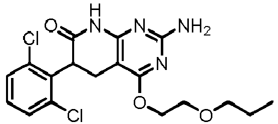

¹H NMR (400 MHz, DMSO-*d*₆): δ 10.51 (s, 1H), 7.58 - 7.47 (m, 2H), 7.37 (t, *J* = 8.1 Hz, 1H), 6.42 (s, 2H), 4.63 (dd, *J* = 12.9, 9.2 Hz, 1H), 4.35 - 4.32 (m, 2H), 3.63 (t, *J* = 4.9 Hz, 2H), 3.35 (t, *J* = 6.6 Hz, 2H), 2.93 - 2.79 (m, 2H), 1.46 (hex, *J* = 7.3 Hz, 2H), 0.80 (t, *J* = 7.4 Hz, 3H).

### Example 13: Synthesis of 2-amino-6-(2,6-dichlorophenyl)-4-(2-methoxyethoxy)-5,6-dihydropyrido[2,3-d]pyrimidine-7(8H)-one

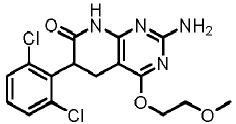

¹H NMR (400 MHz, DMSO-*d*₆): δ 10.54 (s, 1H), 7.55 - 7.48 (m, 2H), 7.37 (t, *J* = 8.1 Hz, 1H), 6.44 (s, 2H), 4.64 (dd, *J* = 12.8, 9.4 Hz, 1H), 4.37 - 4.32 (m, 2H), 3.60 (t, *J* = 4.8 Hz, 2H), 3.26 (s, 3H), 2.93 - 2.79 (m, 2H).

### Example 14: Synthesis of 2-amino-6-(2,6-dichlorophenyl)-4-methoxy-5,6-dihydro pyrido[2,3-d]pyrimidine-7(8H)-one

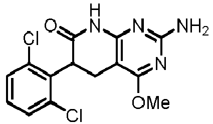

¹H NMR (400 MHz, TFA-d): δ 7.37 (dd, *J* = 15.0, 8.1 Hz, 2H), 7.24 (t, *J* = 8.2 Hz, 1H), 5.08 (dd, *J* = 13.3, 9.4 Hz, 1H), 4.11 (s, 3H), 3.30 - 3.08 (m, 2H).

¹³C NMR (100.6 MHz, TFA-d): δ 173.9, 170.8, 154.3, 146.6, 136.0, 134.7, 131.7, 130.2, 129.4, 128.4, 90.0, 55.7, 42.6, 21.0.

*General procedure for the synthesis of 2-substituted pyrido[2,3-d]pyrimidines*

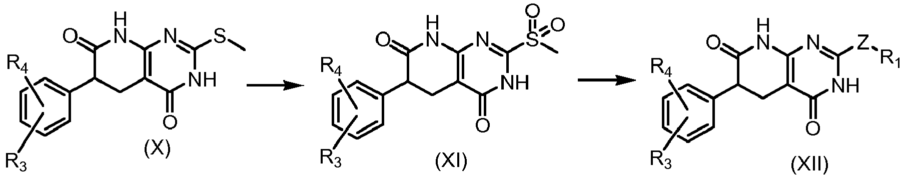

*m*-CPBA (18 mmol) was suspended in 20 mL of DMF and was added dropwise into a solution of the 2-methylthio-substituted pyridopyrimidine (X) (6 mmol), whose synthesis has been previously described [M. Camarasa et al., Mol. Diver. 2013, 17, 525-536], in 20 mL of DMF a 0 ºC. The mixture was stirred overnight a room temperature. Then the solvent was removed by vacuum pressure and the residue was suspended in EtOEt and the solid was collected by filtration and washed with water, and EtOEt to afford the corresponding 2-methylsulphonyl-substituted pyrido[2,3-*d*]pyrimidine (XI). Then, the corresponding amine or alcohol (10 mmol) was added into the solution of 2-methylsulphonyl-substituted pyridopyrimidine (XI)(2 mmol) in 14 mL IPA and the mixture was heated under microwave irradiation at 170 ºC for 5 hours. The solid was collected by filtration and washed with cyclohexane, and Et₂O to afford the corresponding 2-substituted pyrido[2,3-*d*]pyrimidine (XII) as a white solid.

### Example 15: Synthesis of 6-(4-fluorophenyl)-2-(phenylamino)-5,6-dihydropyrido[2,3-d] pyrimidine-4,7(3H,8H)-dione

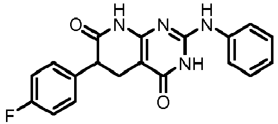

¹H NMR (400 MHz, DMSO-*d*₆): δ 10.47 (s, 2H, 2xNH), 9.25 (s, 1H), 7.79 -7.67 (m, 2H), 7.33 - 7.24 (m, 4H), 7.15 (t, *J* = 8.9 Hz, 2H), 7.04 - 6.98 (m, 1H), 3.85 (dd, *J* = 9.6, 7.0 Hz, 1H), 2.92 - 2.70 (m, 2H).

### Example 16: Synthesis of 6-(2,6-dichlorophenyl)-2-(phenylamino)-5,6-dihydropyrido [2,3-d]pyrimidine-4,7(3H,8H)-dione

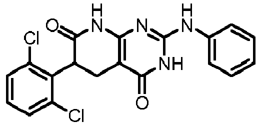

¹H NMR (400 MHz, DMSO-*d*₆): δ 10.56 (s, 2H), 8.87 (s, 1H), 7.72 (d, J = 7.5 Hz, 2H), 7.52 (dd, J = 14.5, 8.0 Hz, 2H), 7.41 - 7.34 (m, 1H), 7.31 (t, J = 7.5 Hz, 2H), 7.03 (t, J = 7.5 Hz, 1H), 4.68 - 4.56 (m, 1H), 2.88 (dd, J = 16.0, 9.0 Hz, 1H), 2.81 - 2.69 (m, 1H).

### Example 17: 6-(2,4-dichlorophenyl)-2-(phenylamino)-5,6-dihydropyrido[2,3-d]pyrimidine-4,7(3H,8H)-dione

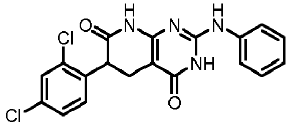

¹H NMR (400 MHz, DMSO-*d*₆): 10.61 (s, 1H), 10.50 (s, 1H), 8.79 (s, 1H), 7.70 (dd, J = 8.7, 1.0 Hz, 2H), 7.64 (d, J = 1.8 Hz, 1H), 7.44 (dd, J = 8.4, 2.0 Hz, 2H), 7.41 (d, J = 8.2 Hz, 1H), 7.30 (dd, J = 8.5, 7.5 Hz, 1H), 7.03 (t, J = 7.4 Hz, 1H), 4.17 (dd, J = 12.4, 7.6 Hz, 1H), 2.88 (dd, J = 15.7, 7.6 Hz, 1H), 2.70 (dd, J = 15.7, 12.4 Hz, 1H).

### Example 18: 6-phenyl-2-(phenylamino)-5,6-dihydropyrido[2,3-d]pyrimidine-4,7(3H,8H)-dione

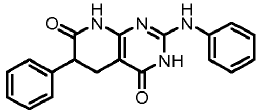

¹H NMR (400 MHz, DMSO-*d*₆): δ 10.48 (s, 2H), 8.80 (s, 1H, NH), 7.73 - 7.66 (m, 2H), 7.34 - 7.22 (m, 7H), 7.02 (tt, J = 7.3, 1.2 Hz, 1H), 3.82 (dd, J = 8.5, 7.1 Hz, 1H), 2.92 - 2.72 (m, 2H).

¹³C NMR (100.6 MHz, DMSO-*d*₆): δ 172.4, 139.7, 139.1, 129.2, 128.8, 128.8, 128.5, 128.4, 127.3, 123.0, 119.8, 114.3, 90.7, 46.2, 25.0.

### Example 19: 6-(2,4-difluorophenyl)-2-(phenylamino)-5,6-dihydropyrido[2,3-d]pyrimidine-4,7(3H,8H)-dione

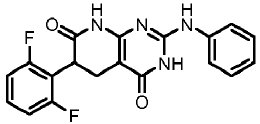

¹H NMR (400 MHz, DMSO-*d*₆): δ 10.58 (s, 2H), 8.83 (s, 1H), 7.74 - 7.68 (m, 2H), 7.46 -7.40 (m, 1H), 7.34 - 7.27 (m, 2H), 7.13 (t, *J* = 8.8 Hz, 2H), 7.03 (t, *J* = 7.4 Hz, 1H), 4.19 (dd, *J* = 14.1, 7.7 Hz, 1H), 2.90 (dd, *J* = 15.7, 7.8 Hz, 1H), 2.53 - 2.55 (m, 1H).

¹³C NMR (100.6 MHz, DMSO-*d*₆): δ 170.0, 162.6, 160.8 (d, *J* = 246.7 Hz), 160.7 (d, *J* = 246.8 Hz), 155.5, 138.6, 129.7 (t, *J* = 10.3 Hz), 128.8, 122.7, 119.4, 115.2 (t, *J* = 18.6 Hz), 113.9, 111.7 (d, *J* = 22.4, 3.2 Hz), 90.1, 36.4, 23.3.

### Example 20: 6-(naphthalen-1-yl)-2-(phenylamino)-5,6-dihydropyrido[2,3-d]pyrimidine-4,7(3H,8H)-dione

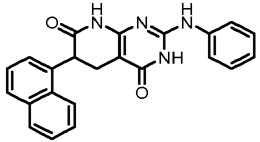

¹H NMR (400 MHz, DMSO-*d*₆): δ 10.64 (s, 1H), 8.86 (s, 1H), 8.09 (d, *J* = 7.7 Hz, 1H,), 7.98 - 7.93 (m, 1H), 7.85 (d, *J* = 8.1 Hz, 1H), 7.74 (d, *J* = 7.7 Hz, 2H), 7.60 - 7.50 (m, 3H), 7.46 (dd, *J* = 8.2, 7.1 Hz, 1H), 7.41- 7.29 (m, 3H), 7.04 (t, *J* = 7.3 Hz, 1H), 4.64 (dd, *J* = 9.1, 7.6 Hz, 1H), 3.00 (dd, *J* = 16.1, 7.7 Hz, 1H), 2.85 (dd, *J* = 16.1, 9.1 Hz, 1H).

¹³C NMR (100.6 MHz, DMSO-*d*₆): δ 172.0, 171.0, 162.9, 155.4, 138.7, 135.9, 133.7, 131.0, 128.8 (C22,C16), 127.5 (C14), 126.1, 125.6, 125.4 (C13), 125.1 (C12), 123.9 (C19), 122.6 (C23), 119.4 (C21), 90.3 (C5), 42.5 (C7), 24.8 (C6).

### Example 21: 6-(4-fluorophenyl)-2-((4-methoxyphenyl)amino)-5,6-dihydropyrido[2,3-d]pyrimidine-4,7(3H,8H)-dione

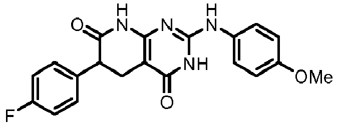

¹H NMR (400 MHz, DMSO-*d*₆): δ 10.40 (s, 1H), 8.62 (s, 1H), 7.56 (d, *J* = 9.0 Hz, 2H), 7.28 (dd, *J* = 8.7, 5.6 Hz, 2H), 7.15 (t, *J* = 8.9 Hz, 2H), 6.87 (d, *J* = 9.0 Hz, 2H), 3.83 (dd, *J* = 9.5, 7.0 Hz, 1H), 3.73 (s, 3H), 2.89 - 2.69 (m, 2H).

### Example 22: Synthesis of 2-(benzylamino)-6-(2,6-dichlorophenyl)-5,6-dihydropyrido [2,3-d]pyrimidine-4,7(3H,8H)-dione

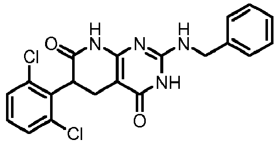

¹H NMR (400 MHz, DMSO-*d*₆): δ 10.57 (s, 1H), 10.27 (s, 1H, NH), 7.50 (ddd, *J* = 16.6, 8.1, 1.3 Hz, 2H), 7.40 - 7.31 (m, 5H), 7.27 (d, *J* = 7.0 Hz, 1H), 6.99 (s, 1H), 4.53 (dd, *J* = 13.4, 8.9 Hz, 1H), 4.47 (d, *J* = 6.1 Hz, 2H), 2.84 - 2.64 (m, 2H).

### Example 23: Synthesis of 6-(4-fluorophenyl)-2-phenoxy-5,6-dihydropyrido[2,3-d] pyrimidine-4,7(3H,8H)-dione

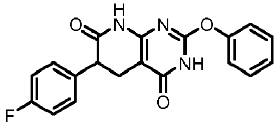

¹H NMR (400 MHz, DMSO-*d*₆): δ 12.58 (s, 1H), 10.48 (s, 1H), 7.44 (dd, *J* = 8.6, 7.3 Hz, 2H), 7.28 - 7.25 (m, 5H), 7.15 (t, *J* = 8.9 Hz, 2H), 3.84 (dd, *J* = 10.0, 7.1 Hz, 1H), 2.88 (dd, *J* = 16.2, 7.1 Hz, 1H), 2.76 (dd, *J* = 16.2, 10.2 Hz, 1H).

### General procedure for the aromatization of pyrido[2,3-d]pyrimidine

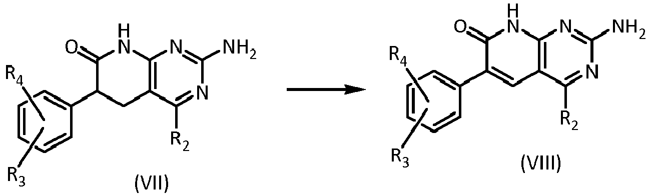

Procedure A: A mixture of (2.7 mmol) pyrido[2,3-*d*]pyrimidine (VII) and (3.5 mmol) of DDQ in 30 mL of 1,2-DCE was heated at 100 ºC overnight protected from moisture. The resulting solution was cooled down, water (300 mL) was added and the resulting precipitate was filtered, washed with EtOH and EtOEt and dried in vacuo over phosphorus pentoxide to afford the corresponding pyrido[2,3-*d*]pyrimidine (VIII) as a brownish solid.

Procedure B: A mixture of (0.5 mmol) pyrido[2,3-*d*]pyrimidine (synthesized as described in I.Pérez et.al., Heterocycles. 2010, 82(1), 581-591 - ) and 60.0 mg (1.5 mmol) of sodium hydride (NaH) (60% dispersion in mineral oil) in 5 mL of anhydrous DMSO was heated for 4 hours at 100 ºC protected from moisture. The resulting solution was cooled down, water (300 mL) was added and it was neutralized with AcOH. The resulting precipitate was filtered, washed with MeOH and EtOEt and dried in vacuo over phosphorus pentoxide to afford the corresponding pyrido[2,3-d]pyrimidine (X) as a brownish solid.

Procedure C: A mixture of (0.5 mmol) pyrido[2,3-*d*]pyrimidine (synthesized as described in Galve et al., Mol. Diver. 2012, 16, 639-649 and 114 mg (1.0 mmol) of activated MnO₂ in 8.5 mL of acetic acid was refluxed for 3 hours. The resulting hot suspension was filtered and the solvent was removed *in vacuo.* The resulting solid was refluxed with water in order to eliminate MnO₂ traces, then filtrated and dried in vacuo over phosphorus pentoxide to afford the corresponding pyrido[2,3-*d*]pyrimidine as a brownish solid.

### Example 24: Synthesis of 2-amino-6-(4-fluorophenyl)-4-((2-propoxyethyl)amino) pyrido[2,3-d]pyrimidine-7(8H)-one

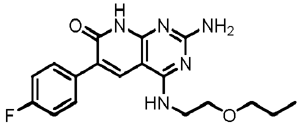

¹H NMR (400 MHz, DMSO-*d*₆): δ 12.26 (s, 1H), 8.30 (s, 1H), 7.83 - 7.73 (m, 2H), 7.23 (t, *J* = 8.7 Hz, 3H), 3.60 - 3.55 (m, 4H), 3.40 - 3.35 (m, 2H), 1.57 - 1.45 (m, 2H), 0.85 (t, J = 7.4 Hz, 3H).

### Example 25: Synthesis of 2-amino-6-(4-fluorophenyl)-4-(pentylamino)pyrido[2,3-d] pyrimidine-7(8H)-one

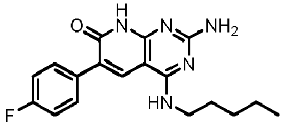

¹H NMR (400 MHz, DMSO-*d*₆): δ 12.11 (s, 1H), 8.27 (s, 1H), 7.76 (m, 2H), 7.24 (m, 2H), 3.47-3.30 (m, 2H) 1.65 - 1.54 (m, 2H), 1.37 - 1.29 (m, 4H), 0.89 (t, *J* = 6.8 Hz, 3H).

### Example 26: Synthesis of 2-amino-6-(4-fluorophenyl)-4-(pyrrolidin-1-yl)pyrido[2,3-d] pyrimidine-7(8H)-one

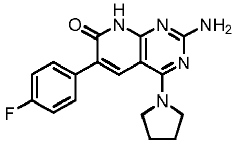

¹H NMR (400 MHz, DMSO-*d*₆): δ 11.77 (s, 1H), 8.10 (s, 1H), 7.73 (m, 2H), 7.19 (t, *J* = 9.0 Hz, 2H), 6.65 (s, 2H), 3.76 (t, *J* = 6.4 Hz, 4H), 1.91 (t, *J* = 6.5 Hz, 4H).

### Example 27: Synthesis of 2-amino-6-(2,6-dichlorophenyl)-4-(2-propoxyethoxy)pyrido [2,3-d]pyrimidine-7(8H)-one

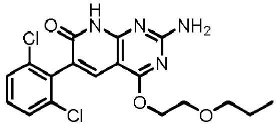

¹H NMR (400 MHz, DMSO-*d*₆): δ 12.00 (s, 1H), 7.58 - 7.51 (m, 3H), 7.42 (dd, *J* = 8.7, 7.4 Hz, 1H), 7.21 (s, 2H), 4.51 - 4.45 (m, 2H), 3.74 - 3.68 (m, 2H), 3.38 (t, *J* = 6.6 Hz, 2H), 1.47 (hex, *J* = 7.3 Hz, 2H), 0.80 (t, *J* = 7.4 Hz, 3H).

### Example 28: 2-amino-6-(2,6-dichlorophenyl)-4-(pentylamino)pyrido[2,3-d]pyrimidine-7(8H)-one

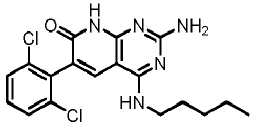

1H NMR (400 MHz, DMSO-d6): δ 10.27 (s, 1H), 8.89 (s, 1H), 8.36 (s, 1H), 7.98 (s, 2H), 7.60 (d, J = 7.8 Hz, 2H), 7.47 (dd, J = 8.8, 7.4 Hz, 1H), 3.49 (q, J = 6.8 Hz, 2H), 1.60 (m, 2H), 1.37 - 1.25 (m, 4H), 0.91 - 0.83 (m, 3H).

### Example 29: Synthesis of 6-(4-fluorophenyl)-2-(phenylamino)pyrido[2,3-d]pyrimidine-4,7(3H,8H)-dione

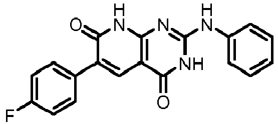

¹H NMR (400 MHz, DMSO-*d*₆): δ 12.18 (s, 1H), 10.81 (s, 1H), 9.12 (s, 1H), 7.89 (s, 1H), 7.78 - 7.72 (m, 4H), 7.35 (dd, *J* = 8.6, 7.3 Hz, 2H), 7.21 (t, *J* = 8.9 Hz, 2H), 7.14 - 7.08 (m, 1H).

### Example 30: Synthesis of 6-phenyl-2-(phenylamino)pyrido[2,3-d]pyrimidine-4,7(3H,8H)-dione

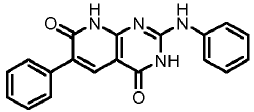

¹H NMR (400 MHz, DMSO-*d*₆): 12.15 (s, 1H), 10.82 (s, 1H), 9.14 (s, 1H), 7.89 (s, 1H), 7.77 (dd, *J* = 8.6, 1.2 Hz, 2H), 7.69 (dd, *J* = 8.3, 1.3 Hz, 2H), 7.42 - 7.29 (m, 5H), 7.10 (t, *J* = 7.4 Hz, 1H).

¹³C NMR (100.6 MHz, DMSO-*d*₆): δ 162.5, 159.9, 155.1, 151.1, 138.0, 136.3, 133.6, 129.0, 128.2, 128.0, 127.2, 124.9, 123.5, 120.2, 97.2.

### Example 31: Synthesis of 6-(4-fluorophenyl)-2-((4-methoxyphenyl)amino)pyrido[2,3-d]pyrimidine-4,7(3H,8H)-dione

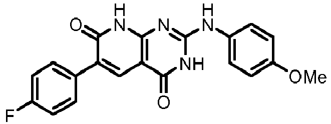

¹H NMR (400 MHz, DMSO-*d*₆): δ 12.07 (s, 1H), 10.74 (s, 1H), 8.96 (s, 1H), 7.87 (s, 1H), 7.74 (dd, *J* = 8.9, 5.6 Hz, 2H), 7.62 (d, *J* = 9.0 Hz, 2H), 7.20 (t, *J* = 8.9 Hz, 2H), 6.92 (d, *J* = 9.0 Hz, 2H), 3.76 (s, 3H).

¹³C NMR (100.6 MHz, DMSO-*d*₆): δ 162.5, 160.1, 159.8, 155.8, 155.3, 151.3, 133.9, 133.6, 132.7 (d, *J* = 2.9 Hz), 130.7, 130.1 (d, *J* = 8.0 Hz), 123.3, 122.4, 114.8 (d, *J* = 21.2 Hz), 114.1, 97.0, 55.3.

### Example 32: Synthesis of 6-(4-fluorophenyl)-2-phenoxy-4-((2-propoxyethyl)amino) pyrido[2,3-d]pyrimidine-7(8H)-one

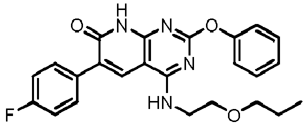

¹H NMR (400 MHz, DMSO-*d*₆): δ 9.08 (s, 1H), 7.64 - 7.58 (m, 3H), 7.43 - 7.37 (m, 2H), 7.25 - 7.17 (m, 3H), 7.07 (t, *J* = 8.7 Hz, 2H), 6.08 (s, 1H), 3.69 (q, *J* = 5.1 Hz, 2H), 3.58 (t, *J* = 5.0 Hz, 2H, 3.42 (t, *J* = 6.7 Hz, 2H), 1.64 - 1.58 (m, 2H), 0.93 (t, *J* = 7.4 Hz, 3H).

### Example 33: Synthesis of 6-(2,6-dichlorophenyl)-2-(phenylamino)-4-((2-propoxyethyl) amino)pyrido[2,3-d]pyrimidine-7(8H)-one

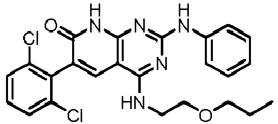

¹H NMR (400 MHz, DMSO-*d*₆) δ 11.85 (s, 1H), 9.34 (s, 1H), 8.09 (s, 1H), 7.87 (m, 3H), 7.56 (m, 2H), 7.44 (dd, *J* = 8.6, 7.6 Hz, 1H), 7.26 (t, *J* = 7.5 Hz, 2H), 6.95 (t, *J* = 7.3 Hz, 1H), 3.67 - 3.54 (m, 4H), 3.36 (t, *J* = 6.7 Hz, 2H), 1.49 (hex, *J* = 7.3 Hz, 2H), 0.83 (t, *J* = 7.4 Hz, 3H).

### Example 34: 4-amino-6-(2,6-difluorophenyl)-2-(phenylamino)pyrido[2,3-d]pyrimidine-7(8H)-one

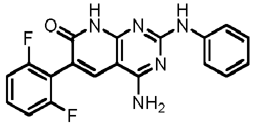

¹H NMR (400 MHz, DMSO-*d*₆) δ 11.87 (br s, 1H), 9.27 (br s, 1H), 8.19 (s, 1H), 7.90 (dd, *J* = 8.7, 1.0 Hz, 2H), 7.51 - 7.43 (m, 1H), 7.35 (br s, 2H), 7.28 - 7.22 (m, 2H), 7.21 - 7.15 (m, 2H), 6.94 (tt, *J* = 7.3, 1.1 Hz, 1H).

¹³C NMR (100.6 MHz, DMSO-d₆) δ 161.5,161.1, 160.3 (dd, *J* = 246.6, 7.3 Hz), 159.6, 156.20, 140.51, 136.63, 130.0 (t, *J* = 10.8 Hz), 128.32, 121.45, 119.53, 113.8 (t, *J* = 20.6 Hz), 113.35, 111.5 (dd, *J* = 18.9, 6.4 Hz), 91.38.

### Example 35: 4-amino-6-(2,6-dichlorophenyl)-2-(phenylamino)pyrido[2,3-d]pyrimidine-7(8H)-one

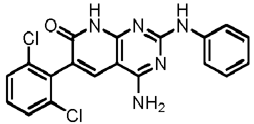

¹H NMR (400 MHz, DMSO-d₆) δ 11.85 (br s, 1H), 9.26 (br s, 1H), 8.04 (s, 1H), 7.88 (d, J = 7.7 Hz, 2H), 7.60 - 7.52 (m, 2H), 7.42 (dd, *J* = 8.7, 7.5 Hz, 1H), 7.29 (br s, 2H), 7.27 - 7.18 (m, 2H), 6.92 (t, *J* = 7.3 Hz, 1H).

¹³C NMR (100.6 MHz, DMSO-d₆) δ 161.3, 161.1, 159.5, 156.1, 140.6, 135.5, 135.4, 135.0, 130.3, 128.3, 128.0, 121.6, 121.4, 119.5, 91.3.

### Example 36: 4-amino-6-(2,6-dibromophenyl)-2-(phenylamino)pyrido[2,3-d]pyrimidine-7(8H)-one

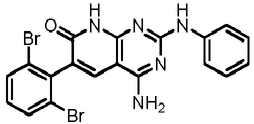

¹H NMR (400 MHz, DMSO-*d*₆): δ 11.85 (br s, 1H), 9.27 (br s, 1H), 8.00 (s, 1H), 7.90 (dd, *J* = 8.7, 1.0 Hz, 2H), 7.76 (d, *J* = 8.1 Hz, 2H), 7.31 (br s, 2H), 7.29 - 7.22 (m, 3H), 6.94 (t, J = 7.3 Hz, 1H).

¹³C NMR (100.6 MHz, DMSO-*d*₆): δ 161.1, 161.0, 159.5, 156.1, 140.6, 138.6, 135.0, 131.7, 131.0, 128.3, 126.0, 125.6, 121.4, 119.5, 91.2.

### Example 37: 4-amino-6-phenyl-2-(phenylamino)pyrido[2,3-d]pyrimidine-7(8H)-one

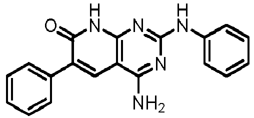

¹H NMR (400 MHz, DMSO-*d*₆) δ 11.76 (br s, 1H), 9.22 (br s, 1H), 8.29 (s, 1H), 7.91 (dd, *J* = 8.7, 1.0 Hz, 2H), 7.75 (dd, *J* = 8.3, 1.3 Hz, 2H), 7.43 - 7.33 (m, 4H), 7.31 (tt, *J* = 7.4, 1.3 Hz, 1H), 7.27 - 7.21 (m, 2H), 6.93 (tt, *J* = 7.3, 1.1 Hz, 1H).

¹³C NMR (100.6 MHz, DMSO-*d*₆) δ 162.7, 161.1, 159.2, 155.3, 140.7, 136.6, 133.0, 128.3, 128.3, 127.8, 126.9, 124.1, 121.3, 119.4, 92.1.

### Example 38: 4-amino-6-(2-fluoro-6-(trifluoromethyl)phenyl)-2-(phenylamino)pyrido [2,3-d]pyrimidine-7(8H)-one

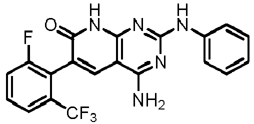

¹H NMR (400 MHz, DMSO-*d*₆) δ 11.85 (br s, 1H), 9.26 (br s, 1H), 8.07 (s, 1H), 7.89 (d, *J* = 8.3 Hz, 2H), 7.65 (m, 4H), 7.32 (br s, 2H), 7.25 (t, *J* = 8.0 Hz, 2H), 6.94 (t, *J* = 7.2 Hz, 1H).

### Example 39: 2,4-diamino-6-(2,6-dichlorophenyl)pyrido[2,3-d]pyrimidine-7(8H)-one

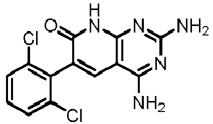

¹H NMR (400 MHz, DMSO-*d*₆) δ 12.17 (br s, 1H), 7.98 (s, 1H), 7.54 (d, *J* = 8.0 Hz, 2H), 7.44 - 7.34 (m, 1H), 7.17 (br s, 2H), 6.81 (br s, 2H).

### Example 40: 6-(2,6-dichlorophenyl)-2-(phenylamino)pyrido[2,3-d]pyrimidine-7(8H)-one

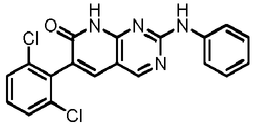

¹H NMR (400 MHz, DMSO-*d6*) δ 12.43 (br s, 1H), 10.08 (br s, 1H), 8.80 (s, 1H), 7.92 (d, *J* = 7.9 Hz, 2H), 7.86 (s, 1H), 7.61 - 7.56 (m, 2H), 7.46 (dd, J = 8.8, 7.4 Hz, 1H), 7.35 - 7.28 (m, 2H), 7.06 - 6.99 (m, 1H).

### General procedure for alkylation of pyrido[2,3-d]pyrimidine at position N8

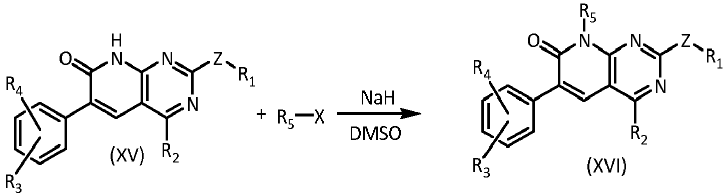

To a solution of (0.7 mmol) of pyrido[2,3-*d*]pyrimidine (XV), whose synthesis has been described above in examples 20-34, in 10 mL of anhydrous DMSO, 28.0 mg (0.7 mmol) of sodium hydride (NaH) (60% dispersion in mineral oil) were added and the mixture was stirred for 1 hour at room temperature under nitrogen atmosphere. After this period, (0.7 mmol) of alkyl halide were added dropwise and then stirred overnight at room temperature. The reaction was quenched by addition of 300 mL of water and the resulting precipitate was filtered, washed with water and dried in vacuo over phosphorus pentoxide to afford the corresponding pyrido[2,3-*d*]pyrimidine (XVI) as a brownish solid.

### Example 41: Synthesis of 2-amino-6-(4-fluorophenyl)-8-methyl-4-((2-propoxyethyl) amino)pyrido[2,3-d]pyrimidine-7(8H)-one

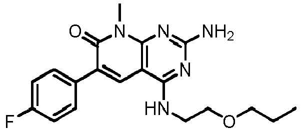

¹H NMR (400 MHz, CDCl₃): δ 7.58 (dd, *J* = 8.7, 5.6 Hz, 2H), 7.47 (s, 1H), 7.05 (t, *J* = 8.8 Hz, 2H), 5.81 (s, 1H), 5.03 (s, 2H), 3.72 (q, *J* = 5.1 Hz, 2H), 3.68 (s, 3H), 3.63 (t, *J* = 5.0 Hz, 2H), 3.44 (t, *J* = 6.7 Hz, 2H), 1.61 (dq, *J* = 14.2, 7.2 Hz, 2H), 0.93 (t, *J* = 7.4 Hz, 3H).

### Example 42: Synthesis of 2-amino-6-(4-fluorophenyl)-8-methyl-4-(pentylamino) pyrido[2,3-d]pyrimidine-7(8H)-one

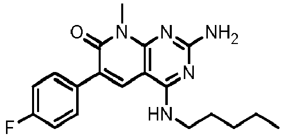

¹H NMR (400 MHz, CDCl₃): δ 7.91 (s, 1H), 7.52 - 7.46 (m, 2H), 6.94 (t, *J* = 8.4 Hz, 2H), 5.64 (t, *J* = 5.4 Hz, 1H), 5.09 (s, 2H), 3.63 (s, 3H), 3.53 - 3.41 (m, 2H), 1.65 - 1.58 (m, 2H), 1.40 - 1.29 (m, 4H), 0.90 (t, *J* = 7.2 Hz, 3H).

### Example 43: Synthesis of 2-amino-6-(4-fluorophenyl)-8-methyl-4-(pentylamino)pyrido [2,3-d]pyrimidine-7(8H)-one

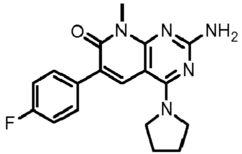

¹H NMR (400 MHz, CDCl₃): δ 8.01 (s, 1H), 7.61 (dd, *J* = 8.8, 5.4 Hz, 2H), 7.08 (t, *J* = 8.8 Hz, 2H), 4.89 (s, 2H), 3.82 - 3.75 (m, 4H), 2.03 - 1.96 (m, 4H).

### Example 44: Synthesis of 2-amino-6-(2,6-dichlorophenyl)-8-methyl-4-(2-propoxy ethoxy)pyrido[2,3-d]pyrimidine-7(8H)-one

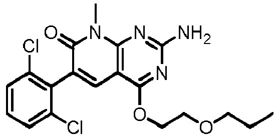

¹H NMR (400 MHz, DMSO-*d*₆): δ 7.62 (s, 1H), 7.54 (d, *J* = 8.0 Hz, 2H), 7.42 (dd, *J* = 8.8, 7.3 Hz, 1H), 7.38 (s, 2H), 4.53 - 4.45 (m, 2H), 3.77 - 3.68 (m, 2H), 3.59 (m, 2H), 3.55 (s, 3H), 1.46 (hex, *J* = 7.1 Hz, 2H), 0.79 (t, *J* = 7.4 Hz, 3H).

### Example 45: Synthesis of 2-amino-6-(2,6-dichlorophenyl)-4-methoxy-8-methyl-5,6-dihydropyrido[2,3-d]pyrimidine-7(8H)-one

This compound was also synthesized following the general procedure described for the alkylation of pyrido[2,3-d]pyrimidine at position N8, but using compound from example 14 as starting material.

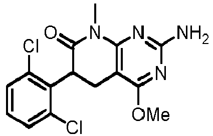

¹H NMR (400 MHz, CD₃OD-*d*₄): δ 7.20 (dd, *J* = 8.7, 5.3 Hz, 2H), 7.02 (t, *J* = 8.8 Hz, 2H), 3.88 (s, 3H), 3.36 (s, 4H), 2.82 - 3.00 (m, 2H).

### Example 46: Synthesis of 6-(2,6-dichlorophenyl)-8-methyl-2-(phenylamino)-4-((2-propoxyethyl)amino)pyrido[2,3-d]pyrimidine-7(8H)-one

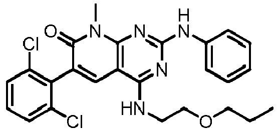

¹H NMR (400 MHz, DMSO-*d*₆) δ 9.52 (s, 1H), 8.14 (s, 1H), 7.93 (s, 1H), 7.83 (d, *J* = 7.6 Hz, 2H), 7.58 (m, 2H), 7.44 (dd, *J* = 8.7, 7.5 Hz, 1H), 7.30 (t, *J* = 7.4 Hz, 2H), 6.98 (t, *J* = 7.3 Hz, 1H), 3.70 - 3.54 (m, 7H), 3.37 (t, *J* = 6.6 Hz, 2H), 1.49 (hex, *J* = 7.4 Hz, 2H), 0.83 (t, *J* = 7.4 Hz, 3H).

¹³C NMR (100.6 MHz, DMSO-*d*₆): 160.8, 160.3, 159.2, 156.2, 140.7, 135.8, 135.8, 133.8, 130.7, 128.9, 128.5, 122.2, 120.4, 120.0, 92.5, 72.2, 68.6, 41.1, 28.9, 22.9, 10.9.

### Example 47: 4-amino-6-(2,6-difluorophenyl)-8-methyl-2-(phenylamino)pyrido[2,3-d]pyrimidine-7(8H)-one

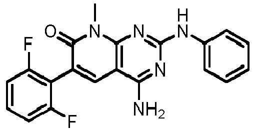

1H NMR (400 MHz, DMSO-d6): δ 9.44 (s, 1H), 8.23 (s, 1H), 7.84 (dd, J = 8.7, 1.0 Hz, 2H), 7.53 - 7.40 (m, 3H), 7.33 - 7.27 (m, 2H), 7.22 - 7.16 (m, 2H), 6.97 (tt, J = 8.2, 1.1 Hz, 1H), 3.61 (s, 3H).

¹³C NMR (100.6 MHz, DMSO-*d*₆): δ 161.64, 161.56 (d, J = 7.4 Hz), 160.8, 159.11 (d, J = 7.3 Hz), 159.11, 156.1, 140.33, 135.01, 130.0 (t, J = 10.0 Hz), 128.4, 121.7, 119.6, 114.2 (t, J = 20.6 Hz), 111.9, 111.5 (dd, J = 18.9, 6.5 Hz), 91.7, 28.4.

### Example 48: 4-amino-6-(2,6-dichlorophenyl)-8-methyl-2-(phenylamino)pyrido[2,3-d]pyrimidine-7(8H)-one

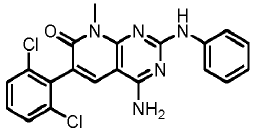

¹H NMR (400 MHz, DMSO-*d*₆) δ 9.41 (br s, 1H), 8.09 (s, 1H), 7.82 (d, *J* = 8.5 Hz, 2H), 7.56 (d, *J* = 7.9 Hz, 2H), 7.42 (t, *J* = 7.6 Hz, 1H), 7.38 (br s, 2H), 7.28 (t, *J* = 7.7 Hz, 2H), 6.95 (t, *J* = 7.3 Hz, 1H), 3.59 (s, 3H).

¹³C NMR (100.6 MHz, DMSO-*d*₆) δ 161.7, 160.5, 159.07, 156.1, 140.4, 135.4, 135.3, 134.0, 130.3, 128.4, 128.0, 121.7, 120.1, 119.6, 91.6, 28.4.

### Example 49: 4-amino-6-(2,6-bromophenyl)-8-methyl-2-(phenylamino)pyrido[2,3-d]pyrimidine-7(8H)-one

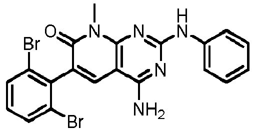

¹H NMR (400 MHz, DMSO-*d*₆): δ 9.43 (br s, 1H), 8.05 (s, 1H), 7.84 (d, *J* = 8.5 Hz, 2H), 7.77 (dd, *J* = 8.0, 0.7 Hz, 2H), 7.40 (br s, 2H), 7.35 - 7.23 (m, 3H), 7.01 - 6.92 (m, 1H), 3.60 (s, 3H).

¹³C NMR (100.6 MHz, DMSO-*d*₆): δ 161.7, 160.3, 159.0, 156.0, 140.4, 138.9, 133.6, 131.7, 131.0, 128.4, 125.6, 124.6, 121.7, 119.6, 91.6, 28.4.

### Example 50: 4-amino-8-methyl-6-phenyl-2-(phenylamino)pyrido[2,3-d]pyrimidine-7(8H)-one

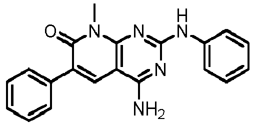

¹H NMR (400 MHz, DMSO-*d*₆) δ 9.36 (br s, 1H), 8.30 (s, 1H), 7.84 (d, *J* = 7.7 Hz, 2H), 7.72 (dd, *J* = 8.5, 1.2 Hz, 2H), 7.41 (t, *J* = 7.6 Hz, 4H), 7.34 - 7.24 (m, 3H), 6.96 (t, *J* = 7.3 Hz, 1H), 3.63 (s, 3H).

¹³C NMR (100.6 MHz, DMSO-*d*₆) δ 161.9, 161.7, 158.8, 155.4, 140.5, 137.1, 131.5, 128.6, 128.5, 127.9, 127.0, 123.0, 121.6, 119.5, 92.4, 28.5.

### Example 51: 4-amino-6-(2-fluoro-6-(trifluoromethyl)phenyl)-8-methyl-2-(phenylamino) pyrido[2,3-d]pyrimidine-7(8H)-one

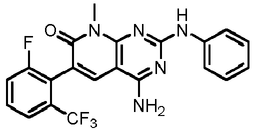

¹H NMR (400 MHz, acetone-*d*₆): δ 8.59 (br s, 1H), 7.99 (s, 1H), 7.91 (dd, *J* = 8.7, 1.0 Hz, 2H), 7.68 - 7.64 (m, 2H), 7.55 - 7.47 (m, 1H), 7.36 - 7.29 (m, 2H), 7.04 - 6.99 (m, 1H), 6.88 (br s 2H), 3.67 (s, 3H).

¹³C NMR (100.6 MHz, acetone-*d*₆): δ 163.0, 162.2, 162.1 (d, *J* = 245.5 Hz), 160.46, 157.73, 141.32, 134.02, 132.2 (dd, *J* = 29.8, 3.1 Hz), 131.1 (d, *J* = 9.0 Hz), 129.37, 125.6 (dq, *J* = 20.7, 1.9 Hz), 124.6 (qd, *J* = 273.8, 3.6 Hz), 122.9, 122.6 (m), 120.6, 120.1 (dd, *J* = 23.4, 0.7 Hz), 117.39, 92.52, 28.81.

### Example 52: 4-amino-6-(2,6-dichlorophenyl)-8-methylpyrido[2,3-d]pyrimidine-7(8H)-one

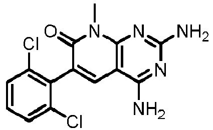

¹H NMR (400 MHz, DMSO-*d*₆) δ 8.00 (s, 1H), 7.55 (d, *J* = 7.9 Hz, 2H), 7.46 - 7.34 (m, 1H), 7.15 (br s, 2H), 6.67 (br s, 2H), 3.52 (s, 3H).

¹³C NMR (100.6 MHz, DMSO-*d*₆) δ 162.8, 162.1, 160.6, 156.7, 135.6, 135.5, 134.1, 130.1, 128.0, 118.4, 90.5, 27.9.

### Example 53: 6-(2,6-dichlorophenyl)-8-methyl-2-(phenylamino)pyrido[2,3-d]pyrimidine-7(8H)-one

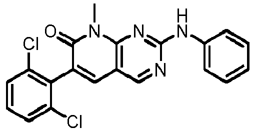

¹H NMR (400 MHz, CDCl₃) δ 8.59 (br s, 1H), 7.71 (dd, *J* = 8.6, 0.9 Hz, 2H), 7.53 (s, 1H), 7.44 - 7.37 (m, 4H), 7.30 - 7.24 (m, 2H), 7.17 - 7.10 (tt, 7.4, 1.0 Hz), 3.81 (s, 3H).

¹³C NMR (100.6 MHz, CDCl₃) δ 161.3, 159.1, 158.4, 155.9, 138.5, 136.2, 135.7, 134.2, 129.9, 129.0, 128.0, 126.1, 123.6, 119.8, 106.6, 28.6.

### Example 54: 4-amino-6-(2,6-dichlorophenyl)-8-ethyl-2-(phenylamino)pyrido[2,3-d] pyrimidine-7(8H)-one

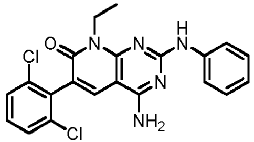

¹H NMR (400 MHz, DMSO-*d*₆) δ 9.41 (br s, 1H), 8.09 (s, 1H), 7.83 (dd, *J* = 8.7, 1.0 Hz, 2H), 7.60 - 7.55 (m, 2H), 7.44 (dd, *J* = 8.7, 7.5 Hz, 1H), 7.38 (br s, 2H), 7.29 (dd, *J* = 8.6, 7.4 Hz, 2H), 6.97 (tt, *J* = 7.3, 1.1 Hz, 1H), 4.35 (q, *J* = 6.9 Hz, 2H), 1.24 (t, *J* = 7.0 Hz, 3H).

¹³C NMR (100.6 MHz, DMSO-*d*₆) δ 161.7, 160.0, 159.2, 155.5, 140.4, 135.3, 135.3, 134.0, 130.2, 128.4, 128.0, 121.6, 120.4, 119.5, 91.6, 36.0, 13.0.

### Example 55: 4-amino-6-(2,6-dichlorophenyl)-2-(phenylamino)-8-propylpyrido[2,3-d]

### pyrimidine-7(8H)-one

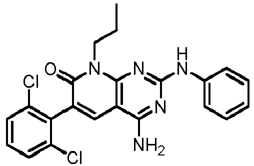

¹H NMR (400 MHz, DMSO-*d*₆) δ 9.39 (br s, 1H), 8.09 (s, 1H), 7.82 (dd, *J* = 8.7, 1.0 Hz, 2H), 7.61 - 7.54 (m, 2H), 7.43 (dd, *J* = 8.7, 7.5 Hz, 1H), 7.35 (br s, 2H), 7.31 - 7.23 (m, 2H), 6.97 (tt, *J* = 7.3, 1.0 Hz, 1H), 4.31 - 4.21 (m, 2H), 1.76 - 1.64 (hex, *J* = 7.4 Hz, 2H), 0.94 (t, *J* = 7.5 Hz, 3H).

¹³C NMR (100.6 MHz, DMSO-*d*₆) δ 161.6, 160.2, 159.1, 155.6, 140.4, 135.4, 135.3, 133.9, 130.2, 128.3, 128.0, 121.7, 120.4, 119.5, 91.6, 42.4, 20.8, 11.3.

### Example 56: 4-amino-8-butyl-6-(2,6-dichlorophenyl)-2-(phenylamino)pyrido[2,3-d] pyrimidine-7(8H)-one

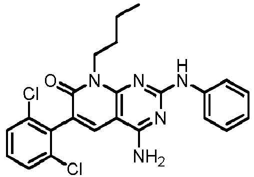

¹H NMR (400 MHz, DMSO-*d*₆) δ 9.39 (br s, 1H), 8.09 (s, 1H), 7.82 (dd, *J* = 8.7, 1.1 Hz, 2H), 7.57 (m, 2H), 7.43 (dd, *J* = 8.7, 7.4 Hz, 1H), 7.34 (br s, 2H), 7.30 - 7.24 (m, 2H), 6.97 (tt, *J* = 7.3, 1.1 Hz, 1H), 4.33 - 4.23 (m, 2H), 1.66 (quint, *J* = 8.0 Hz, 2H), 1.39 (hex, *J* = 7.5 Hz, 2H), 0.93 (t, *J* = 7.4 Hz, 3H).

¹³C NMR (100.6 MHz, DMSO-*d*₆) δ 161.7, 160.3, 159.2, 155.7, 140.4, 135.4, 135.4, 134.0, 130.4, 128.5, 128.1, 121.9, 120.6, 119.6, 91.8, 40.9, 29.7, 20.0, 13.9.

### Example 57: 4-amino-6-(2,6-dichlorophenyl)-8-isobutyl-2-(phenylamino)pyrido[2,3-d] pyrimidine-7(8H)-one

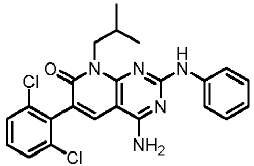

¹H NMR (400 MHz, DMSO-*d*₆) δ 9.38 (br s, 1H), 8.09 (s, 1H), 7.82 (dd, *J* = 8.7, 1.1 Hz, 2H), 7.60 - 7.55 (m, 2H), 7.44 (dd, *J* = 8.7, 7.5 Hz, 1H), 7.34 (br s, 2H), 7.32 - 7.23 (m, 2H), 6.98 (tt, *J* = 7.3, 1.1 Hz, 1H), 4.18 (d, *J* = 7.4 Hz, 2H), 2.29 (hept, *J* = 7.0 Hz, 1H), 0.90 (d, *J* = 6.7 Hz, 6H).

¹³C NMR (100.6 MHz, DMSO-*d*₆) δ 161.7, 160.6, 159.0, 155.9, 140.4, 135.5, 135.4, 133.9, 130.3, 128.4, 128.1, 121.8, 120.6, 119.6, 91.7, 47.4, 26.9, 20.0.

### Example 58: 4-amino-6-(2,6-dichlorophenyl)-8-(2-methoxyethyl)-2-(phenylamino) pyrido[2,3-d]pyrimidine-7(8H)-one

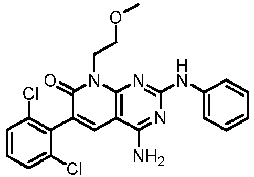

¹H NMR (400 MHz, DMSO-*d*₆) δ 9.42 (br s, 1H), 8.10 (s, 1H), 7.82 (dd, *J* = 8.7, 1.0 Hz, 2H), 7.61 - 7.55 (m, 2H), 7.44 (dd, *J* = 8.7, 7.5 Hz, 1H), 7.40 (br s, 2H), 7.31 - 7.25 (m, 2H), 6.97 (tt, *J* = 7.3, 1.1 Hz, 1H), 4.50 (t, *J* = 6.6 Hz, 2H), 3.61 (t, *J* = 6.6 Hz, 2H), 3.27 (s, 3H).

¹³C NMR (100.6 MHz, DMSO-*d*₆) δ 161.8, 160.3, 159.1, 155.9, 140.3, 135.4, 135.3, 134.3, 130.4, 128.5, 128.1, 121.9, 120.3, 119.7, 91.7, 68.5, 58.2, 40.2.

### Example 59: 4-amino-6-(2,6-dichlorophenyl)-8-(2-hydroxyethyl)-2-(phenylamino) pyrido[2,3-d]pyrimidine-7(8H)-one

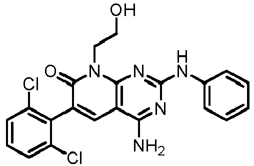

¹H NMR (400 MHz, DMSO-*d*₆) δ 9.40 (br s, 1H), 8.09 (s, 1H), 7.84 (dd, *J* = 8.7, 1.0 Hz, 2H), 7.60 - 7.55 (m, 2H), 7.44 (dd, *J* = 8.7, 7.5 Hz, 1H), 7.38 (br s, 2H), 7.28 (m, 2H), 6.96 (tt, *J* = 7.3, 1.1 Hz, 1H), 4.82 (br t, *J* = 5.3 Hz, 1H), 4.42 (t, *J* = 7.1 Hz, 2H), 3.64 (q, *J* = 6.5 Hz, 2H).

¹³C NMR (100.6 MHz, DMSO-*d*₆) δ 162.1, 160.8, 159.5, 156.3, 140.7, 135.8, 135.7, 134.8, 130.7, 128.9, 128.5, 122.1, 120.7, 119.9, 92.0, 58.3, 43.1.

### Example 60: 4-amino-2-((4-bromophenyl)amino)-6-(2,6-dichlorophenyl)-8-methylpyrido[2,3-d]pyrimidine-7(8H)-one

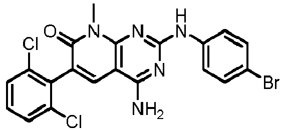

A dispersion of the compound obtained in Example 42 (1.0 mmol) in 40 mL of acetic acid is treated with 0.50 mL (1.0 mmol) of bromine 2M in acetic acid solution for 2 hours at room temperature. The resulting solution is diluted with 1,4-dioxane (40 mL) and the solvent is removed by vacuum azeotropic distillation. A second 1,4-dioxane addition (40 mL) and vacuum distillation yields a white solid that is dispersed in water with ultrasound and mechanical stirring. And the resulting precipitate was filtered, washed with water and dried in vacuo over phosphorus pentoxide to afford the title compound as a brownish solid.

¹H NMR (400 MHz, DMSO-*d*₆) δ 9.60 (br s, 1H), 8.12 (s, 1H), 7.82 (d, *J* = 9.0 Hz, 2H), 7.58 (d, *J* = 8.0 Hz, 2H) 7.57 (br s, 2H), 7.50 - 7.42 (m, 3H), 3.60 (s, 3H).

¹³C NMR (100.6 MHz, DMSO-*d*₆) δ 161.1, 160.4, 158.1, 155.9, 139.5, 135.3, 135.1, 133.9, 131.2, 130.4, 128.1, 121.6, 120.6, 113.4, 91.7, 28.5.

### Example 61: 4-amino-6-(2,6-dichlorophenyl)-8-methyl-2-((4-(methylpiperazin-1-yl)phenyl)aminopyrido[2,3-d]pyrimidine-7(8H)-one

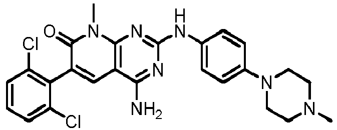

A mixture of the compound obtained in Example 42 (0.4 mmol), 92.0 mg (0.8 mmol) of L-proline, 76.0 mg (0.4 mmol) of copper iodide, 63.4 mg (0.46 mmol) of potassium carbonate and (7.82 mmol) of amine in 4.6 mL of DMSO was heated in microwave for 16 hours at 80 ºC. The resulting solution was cooled down and water (400 mL) was added. The resulting precipitate was filtered, washed with water and dried *in vacuo* over phosphorus pentoxide to afford title compound as a brownish solid.

¹H NMR (400 MHz, DMSO-*d*₆) δ 9.21 (br s, 1H), 8.07 (s, 1H), 7.64 (d, *J* = 9.1 Hz, 2H), 7.60 - 7.55 (m, 2H), 7.43 (dd, *J* = 8.7, 7.5 Hz, 1H), 7.30 (br s, 2H), 6.89 (d, *J* = 9.1 Hz, 2H), 3.58 (s, 3H), 3.12 - 3.03 (m, 4H), 2.48 - 2.41 (m, 4H), 2.22 (s, 3H).

¹³C NMR (100.6 MHz, DMSO-*d*₆) δ 161.6, 160.5, 159.1, 156.1, 146.4, 135.4, 134.0, 132.3, 130.2, 128.4, 128.0, 120.9, 119.5, 115.7, 91.4, 54.7, 48.8, 45.8, 28.3.

### Biological Testing

### Cell lines and cell culture

To analyze the antiviral activity of the compounds, we used a stable cell line, LucUbiNeo-ET, that contains a subgenomic HCV genotype 1b replicon, I389/NS3-3'/ LucUbiNeo-ET, that express luciferase constitutively (Krieger et al, JVI 2001; 75:4614; Frese et al., JGV 2003; 84: 1253-1259). To evaluate the cellular toxicity, we use other cell line, Huh-7/Lunet (Friebe et al., JVI 2005; 79: 380-392), that originally carried a selectable luciferase HCV 1b replicon and was cured by treatment with an HCV-specific inhibitor. These cells are characterized by high permissiveness for HCV RNA replication. LucUbiNeo-ET cell line was cultured at 37°C in a humidified atmosphere with 5% CO₂ in Dulbecco's modified Eagle medium (D-MEM) supplemented with high glucose concentration (4.5g/l) (Invitrogen), 10% (vol/vol) heat-inactivated fetal bovine serum (Hyclone, Cultek), 2mM L-Glutamine (Sigma-aldrich), 1mM sodium pyruvate (Sigma-aldrich), 1% MEM NEEA (Sigma-aldrich), and 0.5 mg/ml of Geneticin (G418) (Gibco, Invitrogen). Cured or Huh-7/Lunet cell line was maintained at the same conditions as described above without the addition of Geneticin.

### Evaluation of the in vitro antiviral activity (EC₅₀) and cytotoxicity assays (CC₅₀)

Antiviral activity was carried out in white-clear bottom 96-well plates, and cytotoxicity assays were performed in clear 96-well plates. Cells were seeded at a density of 10⁴/well in 100ul D-MEM without selection of antibiotics. After 24h, nine fivefold serial dilution of compounds, with 3 replicates at each dilution, were prepared in D-MEM and added to the appropriate wells, yielding concentrations of 125 to 0,00032 ug/ml in a final volume of 100 ul of D-MEM. Following 2 days of incubation, we determined the antiviral activity (EC₅₀) by quantifying the luciferase with the Luciferase Bright Glo assay (Promega). Briefly, LucUbiNeo-ET cells were harvested with the addition of 100 ul of luciferase per well, and after 2 minutes of shaking, the light was read in a Luminometer Fluoroskan Ascent FL. Toxicity (CC₅₀) was analysed in the Lunet/HuH7 cells by the addition of 10 ul of MTT (3-(4,5-dimethylthiazol-2-yl)-2,5-diphenyltetrazolium bromide) per well. After 4h of incubation at 37°C in a humidified atmosphere with 5% CO₂, the amount of formazan produced was quantified spectrophotometrically at 550/620 nm [C. Pannecouque et al, Nature protocol 2008; 3 (3): 427-434]. The EC₅₀ and CC₅₀ values were calculated for each compound. These assays were done in duplicate and the results shown in Table 1 represent the mean value of these duplicates.

Table 1 below shows EC₅₀ (50% effective concentration or concentration that protects 50% of the cell monolayer from virus) and CC₅₀ (50% cytostatic concentration or concentration that results in 50% inhibition of cell growth) value ranges of compounds tested in this assay. Results in table 1 expressed by the following data:
- "A" means EC₅₀ < 1 µM,
- "B" means 1 µM ≤ EC₅₀ < 5 µM,
- "C" means 5 µM ≤ EC₅₀ < 22 µM,
- "D" means CC₅₀ < 10 µM,
- "E" means 10 µM ≤ CC₅₀ < 25 µM, and
- "F" means CC₅₀ ≥ 25 µM.

**Table 1**

| **Example** | **EC₅₀** | **CC₅₀** |
|---|---|---|
| 3 | C | F |
| 4 | B | E |
| 6 | B | D |
| 11 | C | F |
| 13 | C | F |
| 18 | B | F |
| 19 | A | D |
| 20 | C | E |
| 22 | C | F |
| 24 | B | F |
| 28 | B | E |
| 29 | A | E |
| 30 | C | F |
| 31 | C | F |
| 32 | B | F |
| 34 | C | D |
| 36 | C | F |
| 37 | A | E |
| 41 | B | F |
| 42 | C | E |
| 43 | C | F |
| 44 | C | F |
| 45 | C | F |
| 46 | C | F |
| 47 | A | E |
| 48 | A | D |
| 49 | A | D |
| 50 | A | E |
| 51 | B | D |
| 52 | C | F |
| 53 | A | D |
| 54 | B | F |
| 55 | B | F |
| 56 | C | F |
| 57 | A | F |
| 58 | A | F |
| 59 | B | F |
| 60 | A | D |
| 61 | A | D |

## Claims

1. A compound of formula (I): 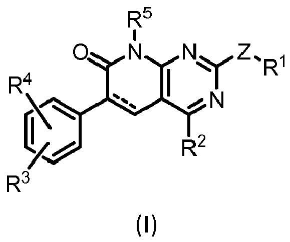 wherein
R¹ is selected from the group consisting of
- H;
- C₆-C₁₄ aryl optionally substituted with one, two or three substituents independently selected from the group consisting of C₁-C₆alkyl, C₁-C₆alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl; and
- C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl;
R² is selected from the group consisting of H; -NR⁶R⁷; and -OR⁸;
where R⁶, R⁷ and R⁸ are independently selected from the group consisting of H, C₁-C₆ alkyl and C₁-C₆ alkyl-O-C₁-C₆ alkylene, and aromatic, saturated or partially saturated 3- to 8-membered monocyclic or 5- to 12-membered bicyclic heterocyclyl which contains from 1 to 4 heteroatoms in the ring system independently selected from the group consisting of N, O, and S, said heterocyclyl being optionally substituted with C₁-C₆alkyl;
or R⁶ and R⁷ together with the nitrogen atom to which they are attached form a saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl;
R³ and R⁴ are independently selected from the group consisting of H, F, Cl, Br, SO₂NH₂ and CF₃;
or R³ and R⁴ together with the phenyl ring to which they are attached, form a C₁₀-C₁₄ bicyclic or tricyclic aryl optionally substituted with one, two or three substituents independently selected from the group consisting of H, F, Cl, Br and CF₃;
R⁵ is selected from the group consisting of
- H;
- C₁-C₆ alkyl optionally substituted with one, two or three substituents independently selected from the group consisting of hydroxy; C₁-C₆ alkoxy, C₁-C₆alkylthio, di(C₁-C₆alkyl)amino, C₁-C₆alkoxycarbonyl, cyano, and F;
- C₃-C₈ cycloalkyl optionally substituted with one, two or three substituents independently selected from the group consisting of hydroxy; C₁-C₆ alkoxy, C₁-C₆alkylthio, C₁-C₆alkoxycarbonyl, di(C₁-C₆alkyl)amino, cyano, and F;
- C₃-C₈ cycloalkyl-C₁-C₃ alkylene, wherein the cycloalkyl is optionally substituted with one, two or three substituents independently selected from the group consisting of hydroxy; C₁-C₆alkoxy, C₁-C₆alkylthio, C₁-C₆alkoxycarbonyl, di(C₁-C₆ alkyl)amino, cyano, and F;
- C₆-C₁₄ aryl optionally substituted with one, two or three substituents independently selected from the group consisting of C₁-C₆alkyl, C₁-C₆alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylsulfonyl, C₁-C₆ alkylsulfinyl di(C₁-C₆ alkyl)aminocarbonyloxy, di(C₁-C₆ alkyl)amino- C₁-C₆ alkyleneoxy, C₁-C₆ alkoxycarbonyl, and C₁-C₆alkoxy-C₁-C₆alkyleneoxy;
- C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆alkoxycarbonyl, and C₁-C₆alkoxy-C₁-C₆alkyleneoxy;
- 3- to 8-membered monocyclic or 5- to 12-membered bicyclic saturated or partially saturated heterocyclyl which contains from 1 to 4 heteroatoms in the ring independently selected from the group consisting of N, O, and S, said heterocyclyl being optionally substituted with one, two or three substituents independently selected from the group consisting of hydroxy; C₁-C₆ alkoxy, C₁-C₆alkylthio, C₁-C₆alkoxycarbonyl, di(C₁-C₆alkyl)amino, cyano, and F;
- heterocyclyl-C₁-C₃ alkylene, wherein the heterocyclyl is a 3- to 8-membered monocyclic or 5- to 12-membered bicyclic saturated or partially saturated ring system which contains from 1 to 4 heteroatoms in the ring independently selected from the group consisting of N, O, and S as defined above, said heterocyclyl being optionally substituted with one, two or three substituents independently selected from the group consisting of hydroxy; C₁-C₆ alkoxy, C₁-C₆alkylthio, C₁-C₆alkoxycarbonyl, di(C₁-C₆alkyl)amino, cyano, and F;
- 5- to 6-membered monocyclic or 8- to 14-membered bicyclic heteroaryl which contains from 1 to 4 heteroatoms in the ring system independently selected from the group consisting of N, O, and S, and wherein the heteroaryl is optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆ alkoxycarbonyl, and C₁-C₆alkoxy-C₁-C₆alkyleneoxy; and
- heteroaryl-C₁-C₃ alkylene, wherein the heteroaryl is a 5- to 6-membered monocyclic or 8- to 14-membered bicyclic ring system which contains from 1 to 4 heteroatoms in the ring independently selected from the group consisting of N, O, and S, said heteroaryl being optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino- C₁-C₆ alkyleneoxy, C₁-C₆alkoxycarbonyl, and C₁-C₆alkoxy-C₁-C₆alkyleneoxy;
Z is selected from the group consisting of NH and O; and
the dotted line represents a bond that may be present or not;
or a stereoisomer, tautomer or pharmaceutically acceptable salt thereof,
for use in the treatment or prevention of infections caused by viruses of the *Flaviviridae* family selected from the group consisting of hepatitis C, Dengue fever, Japanese encephalitis, Kyasanur Forest disease, Murray Valley encephalitis, St. Louis encephalitis, Tick-borne encephalitis, West Nile encephalitis and yellow fever.

2. The compound of formula (I) for use according to claim 1, wherein the bond represented by the dotted line is present.

3. The compound of formula (I) for use according to any one of the preceding claims, wherein Z is NH.

4. The compound of formula (I) for use according to any one of the preceding claims, wherein R¹ is phenyl optionally substituted with one, two or three substituents independently selected from the group consisting of C₁-C₃ alkoxy, F, Cl, Br, and piperazinyl optionally substituted with C₁-C₃alkyl.

5. The compound of formula (I) for use according to any one of the preceding claims, wherein R² is selected from the group consisting of H, OH and NH₂.

6. The compound of formula (I) for use according to any one of the preceding claims, wherein R³ and R⁴ are independently selected from the group consisting of H, F, Cl, Br, or R³ and R⁴ together with the phenyl ring to which they are attached form a naphthyl.

7. The compound of formula (I) for use according to any one of the preceding claims, wherein R⁵ is selected from the group consisting of H, C₁-C₃ alkyl optionally substituted one substituent selected from the group consisting of hydroxyl and C₁-C₃ alkoxy.

8. The compound of formula (I) for use according to claim 1 which is selected from the group consisting of:
- 6-(4-fluorophenyl)-2-(phenylamino)pyrido[2,3-d]pyrimidine-4,7(3H,8H)-dione,
- 4-amino-6-(2,6-difluorophenyl)-8-methyl-2-(phenylamino)pyrido[2,3-d]pyrimidine-7(8H)-one,
- 4-amino-6-(2,6-dichlorophenyl)-8-isobutyl-2-(phenylamino)pyrido[2,3-d] pyrimidine-7(8H)-one,
- 4-amino-2-((4-bromophenyl)amino)-6-(2,6-dichlorophenyl)-8-methylpyrido[2,3-d]pyrimidine-7(8H)-one,
- 6-(2,6-dichlorophenyl)-8-methyl-2-(phenylamino)pyrido[2,3-d]pyrimidine-7(8H)-one,
- 4-amino-6-(2,6-dichlorophenyl)-8-methyl-2-(phenylamino)pyrido[2,3-d] pyrimidine-7(8H)-one,
- 4-amino-6-(2,6-bromophenyl)-8-methyl-2-(phenylamino)pyrido[2,3-d] pyrimidine-7(8H)-one,
- 4-amino-6-phenyl-2-(phenylamino)pyrido[2,3-d]pyrimidine-7(8H)-one,
- 4-amino-8-methyl-6-phenyl-2-(phenylamino)pyrido[2,3-d]pyrimidine-7(8H)-one,
- 4-amino-6-(2,6-dichlorophenyl)-8-methyl-2-((4-(methylpiperazin-1-yl)phenyl) aminopyrido[2,3-d]pyrimidine-7(8H)-one,
- 4-amino-6-(2,6-dichlorophenyl)-8-(2-methoxyethyl)-2-(phenylamino)pyrido [2,3-d]pyrimidine-7(8H)-one,
- 4-((1H-benzo[d][1,2,3]triazol-1-yl)oxy)-6-(4-fluorophenyl)-2-(phenylamino)-3,4,5,6-tetrahydropyrido[2,3-d]pyrimidine-7(8H)-one,
- 4-((1H-benzo[d][1,2,3]triazol-1-yl)oxy)-6-(2,4-dichlorophenyl)-2-(phenylamino)-5,6-dihydropyrido[2,3-d]pyrimidine-7(8H)-one,
- 4-amino-6-(2,6-dichlorophenyl)-2-(phenylamino)-8-propylpyrido[2,3-d] pyrimidine-7(8H)-one,
- 2-amino-6-(4-fluorophenyl)-4-((2-propoxyethyl)amino)pyrido[2,3-d]pyrimidine-7(8H)-one,
- 4-amino-6-(2,6-dichlorophenyl)-8-(2-hydroxyethyl)-2-(phenylamino)pyrido[2,3-d]pyrimidine-7(8H)-one,
- 4-amino-6-(2-fluoro-6-(trifluoromethyl)phenyl)-8-methyl-2-(phenylamino) pyrido[2,3-d]pyrimidine-7(8H)-one,
- 2-amino-6-(2,6-dichlorophenyl)-4-(pentylamino)pyrido[2,3-d]pyrimidine-7(8H)-one,
- 6-(4-fluorophenyl)-2-phenoxy-4-((2-propoxyethyl)amino)pyrido[2,3-d] pyrimidine-7(8H)-one,
- 2-amino-6-(4-fluorophenyl)-8-methyl-4-((2-propoxyethyl)amino)pyrido[2,3-d] pyrimidine-7(8H)-one,
- 6-(2,6-dichlorophenyl)-8-methyl-2-(phenylamino)-4-((2-propoxyethyl)amino) pyrido[2,3-d]pyrimidine-7(8H)-one,
- 4-amino-6-(2,6-dichlorophenyl)-8-ethyl-2-(phenylamino)pyrido[2,3-d] pyrimidine-7(8H)-one,
- 4-amino-6-(2,6-dibromophenyl)-2-(phenylamino)pyrido[2,3-d]pyrimidine-7(8H)-one,
- 2-(benzylamino)-6-(2,6-dichlorophenyl)-5,6-dihydropyrido[2,3-d]pyrimidine-4,7(3H,8H)-dione,
- 2-amino-6-(2,6-dichlorophenyl)-8-methyl-4-(2-propoxyethoxy)pyrido[2,3-d] pyrimidine-7(8H)-one,
- 2-amino-6-(4-fluorophenyl)-8-methyl-4-(pentylamino)pyrido[2,3-d]pyrimidine-7(8H)-one,
- 4-amino-8-butyl-6-(2,6-dichlorophenyl)-2-(phenylamino)pyrido[2,3-d] pyrimidine-7(8H)-one,
- 6-(2,6-dichlorophenyl)-2-(phenylamino)-4-((2-propoxyethyl)amino)-5,6-dihydropyrido[2,3-d]pyrimidine-7(8H)-one,
- 4-amino-6-(2,6-dichlorophenyl)-8-methylpyrido[2,3-d]pyrimidine-7(8H)-one,
- 2-amino-6-(2,6-dichlorophenyl)-4-methoxy-8-methyl-5,6-dihydropyrido[2,3-d] pyrimidine-7(8H)-one,
- 4-amino-6-(2,6-difluorophenyl)-2-(phenylamino)pyrido[2,3-d]pyrimidine-7(8H)-one,
- 4-((1H-benzo[d][1,2,3]triazol-1-yl)oxy)-6-(4-fluorophenyl)-2-phenoxy-3,4,5,6-tetrahydropyrido[2,3-d]pyrimidine-7(8H)-one,
- 2-amino-6-(4-fluorophenyl)-8-methyl-4-(pentylamino)pyrido[2,3-d]pyrimidine-7(8H)-one,
- 2-amino-6-(2,6-dichlorophenyl)-4-(2-methoxyethoxy)-5,6-dihydropyrido[2,3-d] pyrimidine-7(8H)-one,
- 6-(2,6-difluorophenyl)-2-(phenylamino)-5,6-dihydropyrido[2,3-d]pyrimidine-4,7(3H,8H)-dione,
- 6-phenyl-2-(phenylamino)-5,6-dihydropyrido[2,3-d]pyrimidine-4,7(3H,8H)-dione,
- 6-phenyl-2-(phenylamino)-pyrido[2,3-d]pyrimidine-4,7(3H,8H)-dione,
- 6-(naphthalen-1-yl)-2-(phenylamino)-5,6-dihydropyrido[2,3-d]pyrimidine-4,7(3H,8H)-dione, and
- 6-(4-fluorophenyl)-2-((4-methoxyphenyl)amino)pyrido[2,3-d]pyrimidine-4,7(3H,8H)-dione,
or a stereoisomer, tautomer or pharmaceutically acceptable salt thereof.

9. A compound of formula (II): 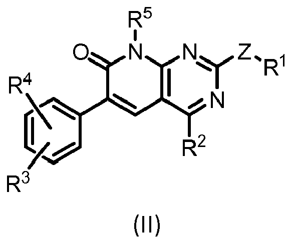 wherein
R¹ is selected from the group consisting of
- H;
- C₆-C₁₄ aryl optionally substituted with one, two or three substituents independently selected from the group consisting of C₁-C₆alkyl, C₁-C₆alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl; and
- C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, SO₂NH₂ and saturated or partially saturated 3- to 8-membered monocyclic heterocyclyl which contains from 1 to 4 heteroatoms in the ring system independently selected from the group consisting of N, O, and S, said heterocyclyl being optionally substituted with C₁-C₆alkyl;
R² is selected from the group consisting of -NR⁶R⁷; and -OR⁸; where R⁶, R⁷ and R⁸ are as defined in claim 1;
R³ and R⁴ are independently selected from the group consisting of H, F, Cl, Br, SO₂NH₂ and CF₃;
or R³ and R⁴ together with the phenyl ring to which they are attached, form a C₉-C₁₄ bicyclic or tricyclic aryl optionally substituted with one, two or three substituents independently selected from the group consisting of H, F, Cl, Br and CF₃;
R⁵ is selected from the group consisting of
- H;
- C₁-C₆ alkyl optionally substituted with one, two or three substituents independently selected from the group consisting of hydroxy; C₁-C₆ alkoxy, C₁-C₆alkylthio, di(C₁-C₆alkyl)amino, C₁-C₆alkoxycarbonyl, cyano, and F;
- C₃-C₈ cycloalkyl optionally substituted with one, two or three substituents independently selected from the group consisting of hydroxy; C₁-C₆ alkoxy, C₁-C₆alkylthio, C₁-C₆alkoxycarbonyl, di(C₁-C₆alkyl)amino, cyano, and F;
- C₃-C₈ cycloalkyl-C₁-C₃ alkylene, wherein the cycloalkyl is optionally substituted with one, two or three substituents independently selected from the group consisting of hydroxy; C₁-C₆alkoxy, C₁-C₆alkylthio, C₁-C₆alkoxycarbonyl, di(C₁-C₆ alkyl)amino, cyano, and F;
- C₆-C₁₄ aryl optionally substituted with one, two or three substituents independently selected from the group consisting of C₁-C₆alkyl, C₁-C₆alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylsulfonyl, C₁-C₆ alkylsulfinyl di(C₁-C₆ alkyl)aminocarbonyloxy, di(C₁-C₆ alkyl)amino- C₁-C₆ alkyleneoxy, C₁-C₆ alkoxycarbonyl, and C₁-C₆alkoxy-C₁-C₆alkyleneoxy;
- C₆-C₁₄ aryl-C₁-C₃ alkylene, wherein the aryl is optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆alkoxycarbonyl, and C₁-C₆alkoxy-C₁-C₆alkyleneoxy;
- 3- to 8-membered monocyclic or 5- to 12-membered bicyclic saturated or partially saturated heterocyclyl which contains from 1 to 4 heteroatoms in the ring independently selected from the group consisting of N, O, and S, said heterocyclyl being optionally substituted with one, two or three substituents independently selected from the group consisting of hydroxy; C₁-C₆ alkoxy, C₁-C₆alkylthio, C₁-C₆alkoxycarbonyl, di(C₁-C₆alkyl)amino, cyano, and F;
- heterocyclyl-C₁-C₃ alkylene, wherein the heterocyclyl is a 3- to 8-membered monocyclic or 5- to 12-membered bicyclic saturated or partially saturated ring system which contains from 1 to 4 heteroatoms in the ring independently selected from the group consisting of N, O, and S as defined above, said heterocyclyl-C₁-C₃ alkyl being optionally substituted with one, two or three substituents independently selected from the group consisting of hydroxy; C₁-C₆ alkoxy, C₁-C₆ alkylthio, C₁-C₆ alkoxycarbonyl, di(C₁-C₆ alkyl)amino, cyano, and F;
- 5- to 6-membered monocyclic or 8- to 14-membered bicyclic heteroaryl which contains from 1 to 4 heteroatoms in the ring system independently selected from the group consisting of N, O, and S, said heteroaryl being optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino-C₁-C₆ alkyleneoxy, C₁-C₆ alkoxycarbonyl, and C₁-C₆alkoxy-C₁-C₆alkyleneoxy; and
- heteroaryl-C₁-C₃ alkylene, wherein the heteroaryl is a 5- to 6-membered monocyclic or 8- to 14-membered bicyclic ring system which contains from 1 to 4 heteroatoms in the ring independently selected from the group consisting of N, O, and S, said heteroaryl being optionally substituted with one, two or three substituents independently selected from the group consisting of C₁-C₆ alkyl, C₁-C₆ alkoxy, C₁-C₆ alkylthio, F, Cl, Br, C₁-C₆ alkoxycarbonyl, C₁-C₆ alkylcarbonyl, C₃-C₈ cycloalkylcarbonyl, C₁-C₆ alkylcarbonyloxy, amino, C₁-C₆ alkylamino, di(C₁-C₆ alkyl)amino, di(C₁-C₆ alkyl)aminocarbonyl, cyano, hydroxyl, hydroxy-C₁-C₆ alkyleneoxy, carboxy, C₁-C₆ alkylcarbonyloxy, C₁-C₆ alkylsulfonyl, C₁-C₆ alkylsulfinyl, di(C₁-C₆ alkyl)aminocarbonyloxy, di(C₁-C₆ alkyl)amino- C₁-C₆ alkyleneoxy, C₁-C₆alkoxycarbonyl, and C₁-C₆alkoxy-C₁-C₆alkyleneoxy;
Z is selected from the group consisting of NH and O;
or a stereoisomer, tautomer or pharmaceutically acceptable salt thereof,
with the proviso that when R³ and R⁴ are both Cl in *ortho* position with respect to the carbon atom bonded to the pyrido[2,3]pyrimidine-7(8H)one ring system, Z is NH, and R¹ is an optionally substituted phenyl, then R² is not NH₂.

10. The compound of formula (II) according to claim 9, wherein R¹ is phenyl optionally substituted with one, two or three substituents independently selected from the group consisting of F, Cl, Br, and piperazinyl optionally substituted with C₁-C₃ alkyl.

11. The compound of formula (II) according to any one of claims 9 or 10, wherein R² is selected from the group consisting of OH and NH₂.

12. The compound of formula (II) according to any one of claims 9 to 11, wherein Z is NH.

13. The compound of formula (II) according to claim 9 which is selected from the group consisting of:
- 6-(4-fluorophenyl)-2-(phenylamino)pyrido[2,3-d]pyrimidine-4,7(3H,8H)-dione,
- 4-amino-6-(2,6-difluorophenyl)-8-methyl-2-(phenylamino)pyrido[2,3-d]pyrimidine-7(8H)-one,
- 4-amino-6-(2,6-bromophenyl)-8-methyl-2-(phenylamino)pyrido[2,3-d] pyrimidine-7(8H)-one,
- 4-amino-6-phenyl-2-(phenylamino)pyrido[2,3-d]pyrimidine-7(8H)-one,
- 4-amino-8-methyl-6-phenyl-2-(phenylamino)pyrido[2,3-d]pyrimidine-7(8H)-one,
- 2-amino-6-(4-fluorophenyl)-4-((2-propoxyethyl)amino)pyrido[2,3-d]pyrimidine-7(8H)-one,
- 4-amino-6-(2-fluoro-6-(trifluoromethyl)phenyl)-8-methyl-2-(phenylamino) pyrido[2,3-d]pyrimidine-7(8H)-one,
- 2-amino-6-(2,6-dichlorophenyl)-4-(pentylamino)pyrido[2,3-d]pyrimidine-7(8H)-one,
- 6-(4-fluorophenyl)-2-phenoxy-4-((2-propoxyethyl)amino)pyrido[2,3-d] pyrimidine-7(8H)-one,
- 2-amino-6-(4-fluorophenyl)-8-methyl-4-((2-propoxyethyl)amino)pyrido[2,3-d] pyrimidine-7(8H)-one,
- 6-(2,6-dichlorophenyl)-8-methyl-2-(phenylamino)-4-((2-propoxyethyl)amino) pyrido[2,3-d]pyrimidine-7(8H)-one,
- 4-amino-6-(2,6-dibromophenyl)-2-(phenylamino)pyrido[2,3-d]pyrimidine-7(8H)-one,
- 2-amino-6-(2,6-dichlorophenyl)-8-methyl-4-(2-propoxyethoxy)pyrido[2,3-d] pyrimidine-7(8H)-one,
- 2-amino-6-(4-fluorophenyl)-8-methyl-4-(pentylamino)pyrido[2,3-d]pyrimidine-7(8H)-one,
- 4-amino-6-(2,6-difluorophenyl)-2-(phenylamino)pyrido[2,3-d]pyrimidine-7(8H)-one,
- 2-amino-6-(4-fluorophenyl)-8-methyl-4-(pentylamino)pyrido[2,3-d]pyrimidine-7(8H)-one,
- 6-phenyl-2-(phenylamino)-pyrido[2,3-d]pyrimidine-4,7(3H,8H)-dione, and
- 6-(4-fluorophenyl)-2-((4-methoxyphenyl)amino)pyrido[2,3-d]pyrimidine-4,7(3H,8H)-dione,
or a stereoisomer, tautomer or pharmaceutically acceptable salt thereof.

14. A compound of formula (II) as defined in any one of claims 9 to 13 or a stereoisomer, tautomer or pharmaceutically acceptable salt thereof, for use as a medicament.

15. A pharmaceutical composition comprising a compound of formula (II) as defined in any one of claims 9 to 13 or a stereoisomer, tautomer or pharmaceutically acceptable salt thereof, and one or more pharmaceutically acceptable excipients.
